# EUROPÄISCHE PATENTSCHRIFT

(11) **EP 3 999 016 B1**
(45) Veröffentlichungstag und Bekanntmachung des Hinweises auf die Patenterteilung: **10.06.2026**
(21) Anmeldenummer: 20739306.7
(22) Anmeldetag: 06.07.2020
(51) Int. Cl.: A61K 8/02, A61K 8/26, A61K 8/58, A61K 8/81, A61Q 5/06

(54) **VERFAHREN ZUM FÄRBEN VON KERATINISCHEM MATERIAL, UMFASSEND DIE ANWENDUNG SILICIUMORGANISCHER VERBINDUNGEN, EINES FARBIGEN EFFEKTPIGMENTS UND EINES FILMBILDENDEN POLYMERS**
METHOD FOR DYEING KERATINOUS MATERIAL, COMPRISING THE USE OF ORGANOSILICON COMPOUNDS, A COLORED EFFECT PIGMENT AND A FILM-FORMING POLYMER
PROCÉDÉ DE COLORATION D'UNE MATIÈRE KÉRATINIQUE COMPRENANT L'APPLICATION DE COMPOSÉS ORGANOSILICIÉS, D'UN PIGMENT À EFFET COLORÉ ET D'UN POLYMÈRE FILMOGÈNE

(30) Priorität: 19.07.2019 DE 102019210691
(43) Veröffentlichungstag der Anmeldung: 25.05.2022
(73) Patentinhaber: Henkel AG & Co. KGaA, 40589 Düsseldorf (DE)
(72) Erfinder: LECHNER, Torsten, 40764 Langenfeld (DE); WESER, Gabriele, 41472 Neuss (DE); KOLONKO, Claudia, 42857 Remscheid (DE); KRIENER, Caroline, 40229 Düsseldorf (DE); SCHUMACHER, Ulrike, 40589 Düsseldorf (DE); NOWOTTNY, Marc, 41069 Mönchengladbach (DE); SCHOEPGENS, Juergen, 41366 Schwalmtal (DE); JAISER, Phillip, 40764 Langenfeld (DE); MATHIASZYK, Carsten, 45277 Essen (DE); HODES, Jing, 58093 Hagen (DE)
(86) Internationale Anmeldenummer: PCT/EP2020/068959
(87) Internationale Veröffentlichungsnummer: WO 2021/013511

(56) Entgegenhaltungen:
- EP-A2- 2 168 633
- WO-A1-2020/035359
- FR-A1- 3 031 672

## Beschreibung

Gegenstand der vorliegenden Anmeldung ist ein Verfahren zum Färben von keratinischem Material, insbesondere von menschlichen Haaren, welches die Anwendung von zwei verschiedenen Mitteln (a), und (b) umfasst. Das Mittel (a) enthält mindestens zwei organische Siliciumverbindungen. Das Mittel (b) enthält mindestens eine farbgebende Verbindung (b2), umfassend mindestens ein ausgewähltes Effektpigment.

Ein zweiter Gegenstand dieser Anmeldung ist eine Mehrkomponenten-Verpackungseinheit (Kit-ofparts) zum Färben von keratinischem Material, insbesondere menschlichen Haaren, welche getrennt voneinander konfektioniert in zwei verschiedenen Containern die Mittel (a) und (b) umfasst.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender eine besonders langanhaltende Färbung seiner Haare, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Siliciumverbindung, hydrophobem Polymer und einem Lösungsmittel auf Haaren Färbungen erzeugen lassen, die gegenüber Shampoonierungen besonders widerstandsfähig sein sollen.

Die WO 2002/05359 A1 beschreibt zweistufige Färbeverfahren mit organischen Siliciumverbindungen, filmbildenden Polymeren und Pigmenten.

Aus der FR 3031672 A1 sind kosmetische Mittel zur Lippenbehandlung bekannt, die Silikonöl und spezielle Mikropartikel umfassen.

Metallische Glanzpigmente oder Metalleffektpigmente finden breite Anwendung in vielen Bereichen der Technik. Sie werden beispielsweise zur Einfärbung von Lacken, Druckfarben, Tinten, Kunststoffen, Gläsern keramischen Produkten und Zubereitungen der dekorativen Kosmetik wie Nagellack eingesetzt. Sie zeichnen sich vor allem durch ihren reizvollen winkelabhängigen Farbeindruck (Goniochromie) und ihren metallartig wirkenden Glanz aus.

Haare mit einem Metallic Finish oder metallischen Reflexen sind im Trend. Durch den Metallic-Ton wirkt das Haar dicker und glänzender.

Es besteht Bedarf, Haarfärbungen mit Effektpigmenten bereitzustellen, die einerseits eine hohe Wasch- und Reibechtheit und andererseits die Haareigenschaften wie Handhabbarkeit und Haptik nicht negativ beeinträchtigen. Dazu wäre es wünschenswert, wenn die eingesetzten Effektpigmente eine hohe Deckkraft aufwiesen und in dünnen Schichten auf dem Haar aufgebracht werden könnten.

Entsprechend war die Aufgabe der vorliegenden Erfindung, ein Färbesystem mit Effektpigmenten bereitzustellen, das mit der oxidativen Färbung vergleichbare Echtheitseigenschaften besitzt. Insbesondere die Waschechtheiten sollten herausragend sein, hierbei sollte jedoch auf den Einsatz der sonst zu diesem Zweck üblicherweise eingesetzten Oxidationsfarbstoffvorprodukte verzichtet werden.

Überraschenderweise hat sich nun herausgestellt, dass die vorgenannte Aufgabe hervorragend gelöst werden kann, wenn keratinische Materialien, insbesondere menschliche Haare, mit einem Verfahren gefärbt werden, bei welchem mindestens zwei Mittel (a) und (b) auf die keratinischen Materialien (Haare) appliziert werden. Hierbei enthält das Mittel (a) mindestens zwei organische Siliciumverbindungen und das Mittel (b) enthält mindestens ein ausgewähltes Effektpigment (b1) und ein filmbildendes Polymer (b2).

Bei Einsatz der zwei Mittel (a) und (b) in einem Färbeverfahren konnte keratinisches Material in besonders hoher Farbintensität und hohen Echtheiten gefärbt werden.

Ein erster Gegenstand der vorliegenden Erfindung ist ein Verfahren zum Färben von keratinischem Material, insbesondere menschlichen Haaren, umfassend die folgenden Schritte:
- Anwendung eines Mittels (a) auf dem keratinischen Material, wobei das Mittel (a) enthält:
   (a1) mindestens eine organische Siliciumverbindung, umfassend mindestens eine organische Siliciumverbindungen der Formel (I) und mindestens eine organische Siliciumverbindungen der Formel (IV) enthält, wobei in der organischen Siliciumverbindung der Formel (I)

      R₁R₂N-L-Si(OR₃)ₐ(R₄)_{b} (I),

      - R₁, R₂ unabhängig voneinander für ein Wasserstoffatom oder eine C₁-C₆-Alkylgruppe stehen,
      - L für eine lineare oder verzweigte, zweiwertge C₁-C₂₀-Alkylengruppe steht,
         - R₃ für ein Wasserstoffatom oder für eine C₁-C₆-Alkylgruppe steht,
      - R₄ für eine C₁-C₆-Alkylgruppe steht,
      - a, für eine ganze Zahl von 1 bis 3 steht, und
      - b für die ganze Zahl 3 - a steht, und
   wobei in der organischen Siliciumverbindung der Formel (IV)

      R₉Si(OR₁₀)ₖ(R₁₁)ₘ (IV),

      - R₉ für eine C₁-C₁₈-Alkylgruppe steht,
      - R₁₀ für ein Wasserstoffatom oder eine C₁-C₆-Alkylgruppe steht,
      - R₁₁ für eine C₁-C₆-Alkylgruppe steht
      - k für eine ganze Zahl von 1 bis 3 steht, und
      - m für die ganze Zahl 3 - k steht, und
- Anwendung eines Mittels (b) auf dem keratinischen Material, wobei das Mittel (b) enthält:
   (b1) mindestens eine farbgebende Verbindung, umfassend mindestens ein Effektpigment, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallpartikelträgerschicht vorgesehen sind, und
   (b2) mindestens ein filmbildendes Polymer.

In den Effektpigmenten werden Interferenzfarben mit hohem Chroma durch Interferenz zwischen dem von der Oberfläche des Substratplättchens reflektierten Licht und dem von den Oberflächen der Metallpartikel reflektierten Licht entwickelt.

Entsprechend können mit Hilfe der "gefärbten" Effektpigmente keratinische Fasern in besonders intensiven und zahlreichen Farbnuancen gefärbt werden.

### Keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

### Mittel (a) und (b)

Im Rahmen des Verfahrens werden die Mittel (a) und (b) auf dem keratinischen Material, insbesondere den menschlichen Haaren, appliziert. Die zwei Mittel (a) und (b) sind voneinander verschieden.

Entsprechend wird ein Verfahren zum Färben von keratinischem Material, insbesondere von menschlichen Haaren, offenbart, umfassend die folgenden Schritte:
- Anwendung eines Mittels (a) auf dem keratinischen Material, wobei das Mittel (a) mindestens eine organische Siliciumverbindungen der Formel (I) und mindestens eine organische Siliciumverbindungen der Formel (IV) enthält, wobei in der organischen Siliciumverbindung der Formel (I)

   R₁R₂N-L-Si(OR₃)ₐ(R₄)_{b} (I),

   - R₁, R₂ unabhängig voneinander für ein Wasserstoffatom oder eine C₁-C₆-Alkylgruppe stehen,
   - L für eine lineare oder verzweigte, zweiwertge C₁-C₂₀-Alkylengruppe steht,
      - R₃ für ein Wasserstoffatom oder für eine C₁-C₆-Alkylgruppe steht,
   - R₄ für eine C₁-C₆-Alkylgruppe steht,
   - a, für eine ganze Zahl von 1 bis 3 steht, und
   - b für die ganze Zahl 3 - a steht, und
   wobei in der organischen Siliciumverbindung der Formel (IV)

   R₉Si(OR₁₀)ₖ(R₁₁)ₘ (IV),

   - R₉ für eine C₁-C₁₈-Alkylgruppe steht,
   - R₁₀ für ein Wasserstoffatom oder eine C₁-C₆-Alkylgruppe steht,
   - R₁₁ für eine C₁-C₆-Alkylgruppe steht
   - k für eine ganze Zahl von 1 bis 3 steht, und
   - m für die ganze Zahl 3 - k steht,, und
- Anwendung eines Mittels (b) auf dem keratinischen Material, wobei das Mittel (b) enthält:
   (b1) mindestens eine farbgebende Verbindung, umfassend mindestens ein Effektpigment, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallpartikelträgerschicht vorgesehen sind, und
   (b2) und mindestens ein filmbildendes Polymer,
wobei die zwei Mittel (a) und (b) voneinander verschieden sind.

### Mittel (a)

Das Mittel (a) ist gekennzeichnet durch seinen Gehalt an mindestens einer organischen Siliciumverbindung, insbesondere mindestens eines organischen Silans. Die im Mittel (a) enthaltenen organischen Siliciumverbindungen bzw. organischen Silane sind reaktive Verbindungen.

Das Mittel (a) enthält die organischen Siliciumverbindung(en), insbesondere das oder die organischen Silane in einem kosmetischen Träger, der wasserhaltig, wasserarm oder auch wasserfrei sein kann. Zudem kann der kosmetische Träger flüssig, gelartig, cremeförmig, pastös, pulverförmig oder auch fest (z.B. in Form einer Tablette oder eines Presslings) sein. Bevorzugt ist der kosmetische Träger des Mittels (a) ein wässriger oder wässrig-alkoholischer Träger. Zum Zwecke der Haarfärbung sind solche Träger beispielsweise Cremes, Emulsionen, Gele oder auch Tensid-haltige schäumende Lösungen, wie beispielsweise Shampoos, Schaumaerosole, Schaumformulierungen oder andere Zubereitungen, die für die Anwendung auf dem Haar geeignet sind.

Der kosmetische Träger ist bevorzugt wasserhaltig, was bedeutet, dass der Träger - bezogen auf sein Gewicht - mindestens 2 Gew.-% Wasser enthält. Bevorzugt liegt der Wassergehalt oberhalb von 5 Gew.-%, weiter bevorzugt oberhalb von 10 Gew.-% noch weiter bevorzugt oberhalb von 15 Gew.-%. Der kosmetische Träger kann auch wässrig-alkoholisch sein. Unter wässrig-alkoholischen Lösungen sind im Sinne der vorliegenden Erfindung wässrige Lösungen enthaltend 2 bis 70 Gew.-% eines C₁-C₄-Alkohols, insbesondere Ethanol bzw. Isopropanol, zu verstehen. Die Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Der Begriff "Mittel zur Färbung" wird im Rahmen dieser Erfindung für eine durch Einsatz von Pigmenten und/oder direktziehenden Farbstoffen hervorgerufene Farbgebung des keratinischen Materials, insbesondere des menschlichen Haares, verwendet. Bei dieser Färbung lagern sich die vorgenannten farbgebenden Verbindungen in einem besonders homogenen und glatten Film an der Oberfläche des keratinischen Materials ab oder diffundieren in die keratinische Faser hinein. Der Film bildet sich in situ durch Oligomerisierung bzw. Polymerisierung des oder der organischen Siliciumverbindungen, sowie durch die Wechselwirkung von organischer Siliciumverbindung mit den farbgebenden Verbindungen.

### Organische Siliciumverbindungen

Als erfindungswesentlichen Bestandteil enthält das Mittel (a) mindestens zwei organische Siliciumverbindungen (a1).

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das SiliciumAtom geknüpft ist. Die organische Siliciumverbindungen sind bevorzugt Verbindungen, die ein bis drei Siliciumatome enthalten. Besonders bevorzugt enthalten die organische Siliciumverbindungen ein oder zwei Siliciumatome.

Das Mittel (a) enthält besonders bevorzugt mindestens zwei organische Siliciumverbindungen (a1), die aus Silanen mit einem, zwei oder drei Siliciumatomen ausgewählt ist.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das Mittel (a) enthält mindestens eine organische Siliciumverbindung (a1) der Formel (I).

Das Verfahren ist dadurch gekennzeichnet, dass auf dem keratinischen Material oder den menschlichen Haaren ein Mittel (a) angewendet wird, wobei das Mittel (a) mindestens eine organische Siliciumverbindung (a1) der Formel (I) enthält,

R₁R₂N-L-Si(OR₃)ₐ(R₄)_{b} (I),

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder eine C₁-C₆-Alkylgruppe steht,
- R₄ für eine C₁-C₆-Alkylgruppe steht
- a, für eine ganze Zahl von 1 bis 3 steht, und

- b für die ganze Zahl 3 - a steht

Die Substituenten R₁, R₂, R₃ und R₄ in den Verbindungen der Formel (I) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen (a1) der Formel (I)

R₁R₂N-L-Si(OR₃)ₐ(R₄)_{b} (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung (a1) befindet sich die Struktureinheit oder der Linker - L- der für eine lineare oder verzweigte, zweiwertige C₁-C₂₀-Alkylengruppe steht.

Bevorzugt steht -L- für eine lineare, zweiwertige C₁-C₂₀-Alkylengruppe. Weiter bevorzugt steht -L- für eine lineare zweiwertige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die organischen Siliciumverbindungen (a1) der Formel (I)

R₁R₂N-L-Si(OR₃)ₐ(R₄)_{b} (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Färbungen mit den besten Waschechtheiten konnten erhalten werden, wenn das Mittel (a) mindestens eine organische Siliciumverbindung (a1) der Formel (I) enthält, in welcher die Reste R₃, R₄ unabhängig voneinander für eine Methylgruppe oder für eine Ethylgruppe stehen.

Weiterhin konnten Färbungen mit den besten Waschechtheiten erhalten werden, wenn das Mittel (a) mindestens eine organische Siliciumverbindung (a1) der Formel (I) enthält, in welcher der Rest a für die Zahl 3 steht. In diesem Fall steht der Rest b für die Zahl 0.

In einer weiteren bevorzugten Ausführungsform ist ein Mittel (a) dadurch gekennzeichnet, dass es mindestens eine organische Siliciumverbindung (a1) der Formel (I) enthält,
wobei
- R₃, R₄ unabhängig voneinander für eine Methylgruppe oder für eine Ethylgruppe stehen und
- a für die Zahl 3 steht und
- b für die Zahl 0 steht.

In einer weiteren bevorzugten Ausführungsform ist das Verfahren dadurch gekennzeichnet, dass das Mittel (a) mindestens eine organische Siliciumverbindung (a1) der Formel (I) enthält,

R₁R₂N-L-Si(OR₃)ₐ(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- R₄ für eine Methylgruppe oder für eine Ethylgruppe steht,
- a für die Zahl 3 steht und
- b für die Zahl 0 steht.

Wenn b für die Zahl 0 steht, kommt der Rest R₄ in den Verbindungen der Formel (I) nicht vor.

In einer weiteren bevorzugten Ausführungsform ist das Verfahren demnach dadurch gekennzeichnet, dass das Mittel (a) mindestens eine organische Siliciumverbindung (a1) der Formel (I) enthält,

R₁R₂N-L-Si(OR₃)ₐ(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- a für die Zahl 3 steht und
- b für die Zahl 0 steht.

Zur Lösung der Aufgabenstellung besonders gut geeignete organische Siliciumverbindungen (a1) der Formel (I) sind

### - (3-Aminopropyl)triethoxysilan

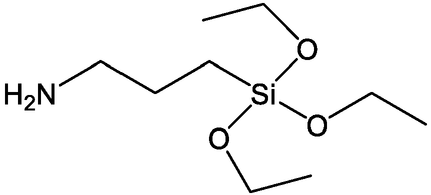

### - (3-Aminopropyl)trimethoxysilan

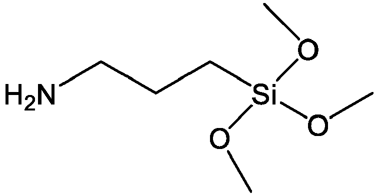

### - 1-(3-Aminopropyl)silantriol

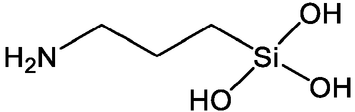

### - (2-Aminoethyl)triethoxysilan

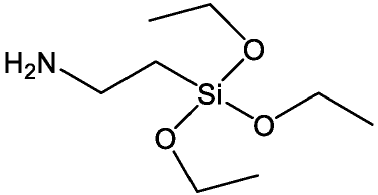

### - (2-Aminoethyl)trimethoxysilan

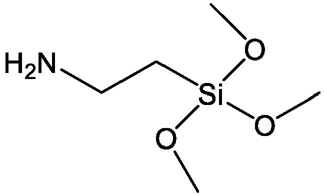

### -1-(2-Aminoethyl)silantriol

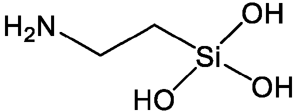

### - (3-Dimethylaminopropyl)triethoxysilan

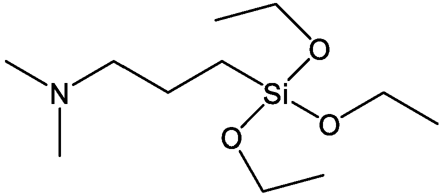

### - (3-Dimethylaminopropyl)trimethoxysilan

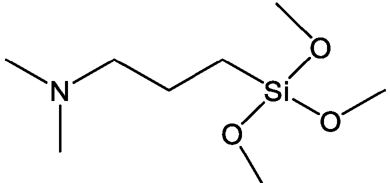

### -1-(3-Dimethylaminopropyl)silantriol

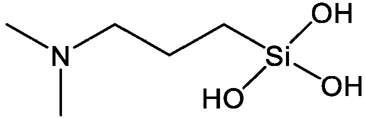

### - (2-Dimethylaminoethyl)triethoxysilan

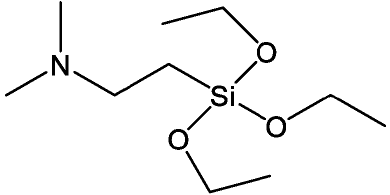

### - (2-Dimethylaminoethyl)trimethoxysilan und/oder

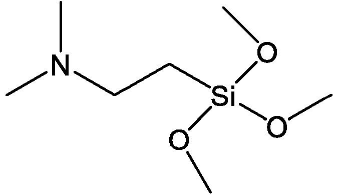

### -1-(2-Dimethylaminoethyl)silantriol

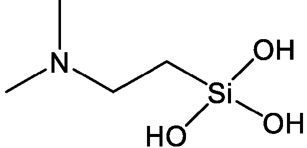

In einer weiteren bevorzugten Ausführungsform ist das Verfahren dadurch gekennzeichnet, dass das Mittel (a) mindestens eine organische Siliciumverbindung (a1) der Formel (I) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan,
- 1-(2-Dimethylaminoethyl)silantriol
und Mischungen daraus.

Die vorgenannten organische Siliciumverbindung (a1) der Formel (I) sind kommerziell erhältlich.

(3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erhältlich.

Im Rahmen einer weiteren Ausführungsform enthält das Mittel (a) ferner mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II).

Die siliciumorganischen Verbindungen (a1) der Formel (II) tragen jeweils an ihren beiden Enden die Silicium-haltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A‴)]ₕ- Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organische Siliciumverbindung (a1) der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Färbungen mit den besten Waschechtheiten konnten erhalten werden, wenn die Reste c und c' beide für die Zahl 3 stehen. In diesem Fall stehen d und d' beide für die Zahl 0.

In einer weiteren bevorzugten Ausführungsform ist das Verfahren dadurch gekennzeichnet, dass das Mittel (a) ferner mindestens eine organische Siliciumverbindung (a1) der Formel (II) enthält,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die organischen Siliciumverbindung der Formel (IIa)

(R₅O)₃Si-(A)ₑ-[NR₇-(A')]_{f}-[O-(A")]_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ-sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der Waschechtheit erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die organischen Siliciumverbindung (a1) der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und Aʺʺ stehen unabhängig voneinander für eine lineare oder verzweigte, zweiwertige C₁-C₂₀-Alkylengruppe. Bevorzugt stehen die Reste A, A', A", A‴ und Aʺʺ unabhängig voneinander für eine lineare, zweiwertige C₁-C₂₀-Alkylengruppe. Weiter bevorzugt stehen die Reste A, A', A", A‴ und Aʺʺ unabhängig voneinander für eine lineare zweiwertige C₁-C₆-Alkylengruppe. Besonders bevorzugt stehen die Reste A, A', A", A‴ und Aʺʺ unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und Aʺʺ für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (III)

-(Aʺʺ)-Si(R₆")_{d}"(OR₅")_{c}" (III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung (a1) die Gruppierung [NR₇-(A')], aber nicht die Gruppierung -[NR₈-(A‴)]. Steht nun der Rest R7 für eine Gruppierung der Formel (III), so enthält das Mittel (a) eine organische Siliciumverbindung mit 3 reaktiven Silan-Gruppen.

In einer weiteren bevorzugten Ausführungsform ist das Verfahren dadurch gekennzeichnet, dass das Mittel (a) ferner mindestens eine organische Siliciumverbindung (a1) der Formel (II) enthält,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen, und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform ist das Verfahren dadurch gekennzeichnet, dass das Mittel (a) ferner mindestens eine organische Siliciumverbindung (a1) der Formel (II) enthält, wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂- ) oder eine Propylengruppe (-CH₂-CH₂-CH₂) stehen, und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der Aufgabenstellung gut geeignete organische Siliciumverbindungen (a1) der Formel (II) sind

### - 3-(Trimethoxysilyl)-N-[3-(trimethoxysilyl)propyl]-1-propanamin

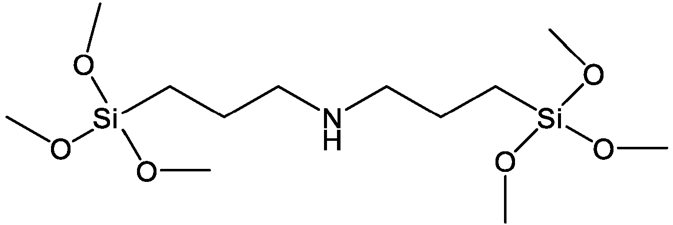

### - 3-(Triethoxysilyl)-N-[3-(triethoxysilyl)propyl]-1-propanamin

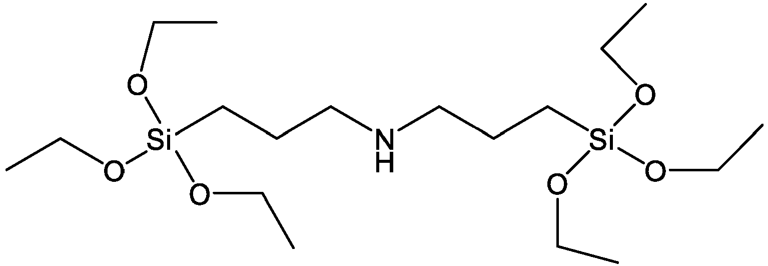

### - N-Methyl-3-(trimethoxysilyl)-N-[3-(trimethoxysilyl)propyl]-1-propanamin

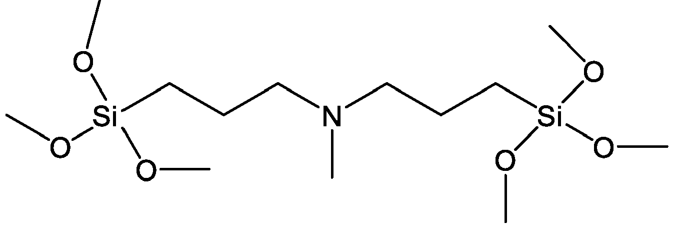

### - N-Methyl-3-(triethoxysilyl)-N-[3-(triethoxysilyl)propyl]-1-propanamin

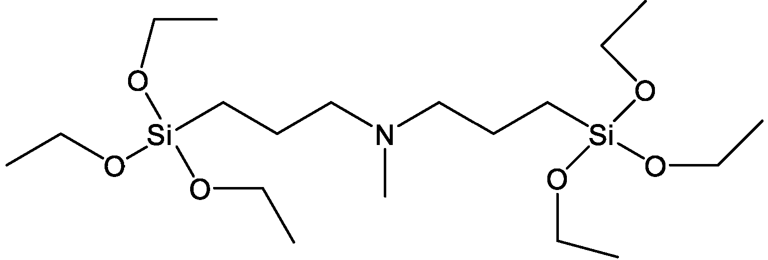

### - 2-[Bis[3-(trimethoxysilyl)propyl]amino]-ethanol

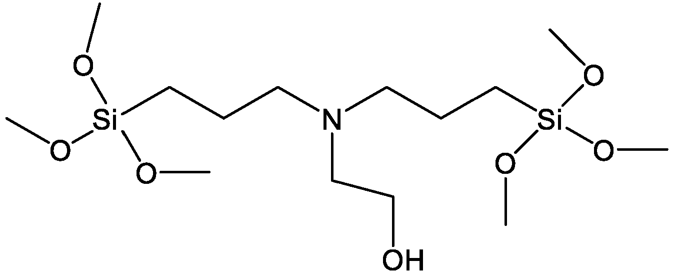

### - 2-[Bis[3-(triethoxysilyl)propyl]amino]-ethanol

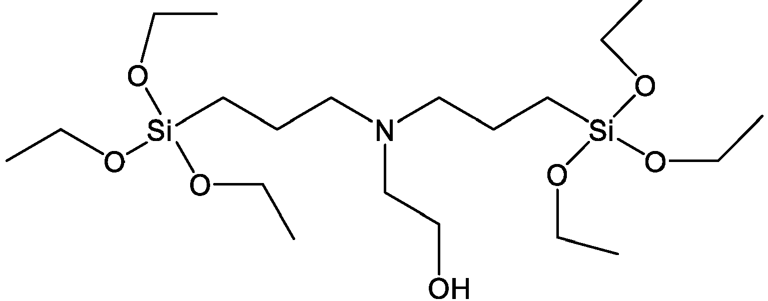

### - 3-(Trimethoxysilyl)-N,N-bis[3-(trimethoxysilyl)propyl]-1-propanamin

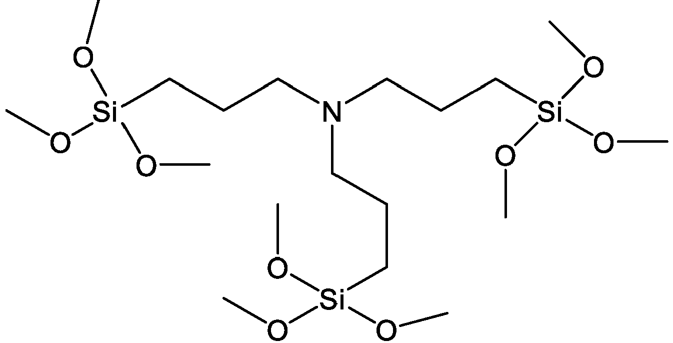

### - 3-(Triethoxysilyl)-N,N-bis[3-(triethoxysilyl)propyl]-1-propanamin

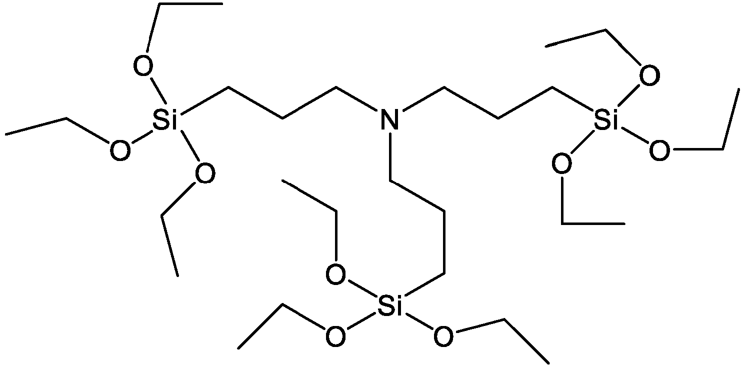

### - N1,N1-Bis[3-(trimethoxysilyl)propyl]-1,2-ethanediamin,

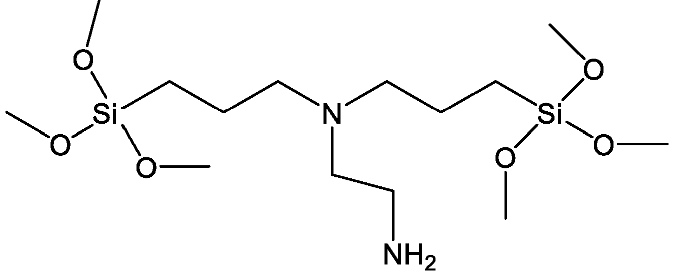

### - N1,N1-Bis[3-(triethoxysilyl)propyl]-1,2-ethanediamin,

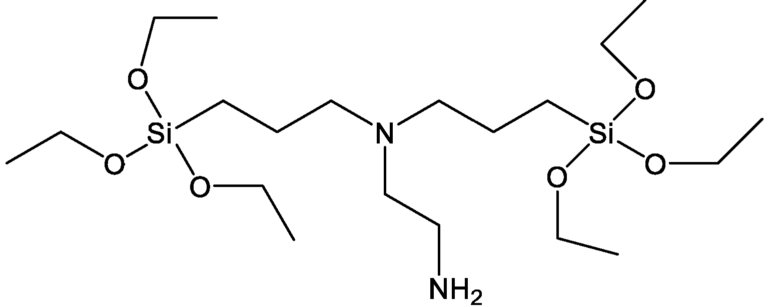

### - N,N-Bis[3-(trimethoxysilyl)propyl]-2-propen-1-amin

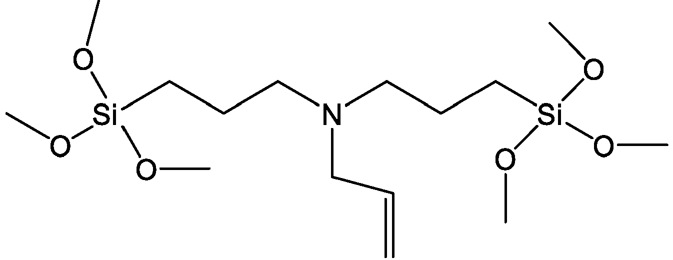

### - N,N-Bis[3-(triethoxysilyl)propyl]-2-propen-1-amin

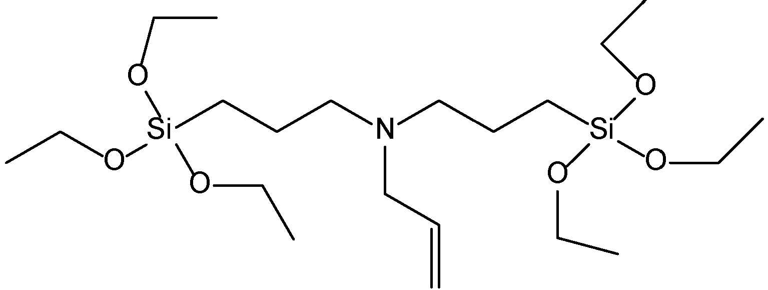

Die vorgenannten organische Siliciumverbindung (a1) der Formel (II) sind kommerziell erhältlich. Bis(trimethoxysilylpropyl)amine mit der CAS-Nummer 82985-35-1 kann beispielsweise von Sigma-Aldrich käuflich erworben werden.

Bis[3-(triethoxysilyl)propyl]amine mit der CAS-Nummer 13497-18-2 kann zum Beispiel von Sigma-Aldrich käuflich erworben werden.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In einer weiteren bevorzugten Ausführungsform ist ein Mittel (a) dadurch gekennzeichnet, dass es mindestens eine organische Siliciumverbindung (a1) der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin.

Das Mittel (a) enthält mindestens eine organische Siliciumverbindung (a1) der Formel (IV)

R₉Si(OR₁₀)ₖ(R₁₁)ₘ (IV).

Die organische(n) Siliciumverbindung(en) (a1) der Formel (IV) kann/können auch als Silane vom Typ der Alkylalkoxysilane oder der Alkylhydroxysilane bezeichnet werden,

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist das Verfahren dadurch gekennzeichnet, dass das Mittel (a) mindestens eine organische Siliciumverbindung (a1) der Formel (IV) enthält.

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist das Verfahren dadurch gekennzeichnet, dass das Mittel (a) zusätzlich zu der oder den organischen Siliciumverbindungen (a1) der Formel (I) mindestens eine weitere organische Siliciumverbindung (a1) der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist das Verfahren dadurch gekennzeichnet, dass das Mittel (a) zusätzlich zu der oder den organischen Siliciumverbindungen (a1) der Formel (II) mindestens eine weitere organische Siliciumverbindung (a1) der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Das Mittel (a) enthält zusätzlich zu der oder den organischen Siliciumverbindungen (a1) der Formel (I) mindestens eine weitere organische Siliciumverbindung (a1) der Formel (IV)

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen (a1) der Formel (IV) steht der Rest R₉ für eine C₁-C₁₈-Alkylgruppe. Diese C₁-C₁₈-Alkylgruppe ist gesättigt und kann linear oder verzweigt sein. Bevorzugt steht R₉ für eine lineare C₁-C₁₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe, eine n-Dodecylgruppe oder eine n-Octadecylgruppe. Besonders bevorzugt steht R₉ für eine Methylgruppe, eine Ethylgruppe, eine n-Propylgruppe, eine n-Hexylgruppe oder eine n-Octylgruppe.

In den organischen Siliciumverbindungen (a1) der Formel (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen (a1) der Form (IV) steht der Rest R₁₁ für eine C₁-C₆-Alkylgruppe. Besonders bevorzugt steht R11 für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Färbungen mit den besten Waschechtheiten konnten erhalten werden, wenn in dem Verfahren ein Mittel (a) eingesetzt wurde, welches mindestens eine organische Siliciumverbindung (a1) der Formel (IV) enthält, in welcher der Rest k für die Zahl 3 steht. In diesem Fall steht der Rest m für die Zahl 0.

Zur Lösung der erfindungsgemäßen Aufgabenstellung besonders gut geeignete organische Siliciumverbindungen (a1) der Formel (IV) sind

### - Methyltrimethoxysilan

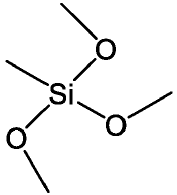

### - Methyltriethoxysilan

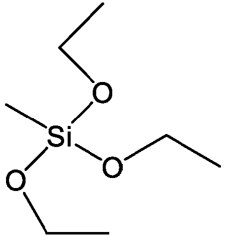

### - Ethyltrimethoxysilan

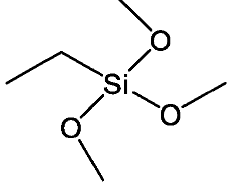

### - Ethyltriethoxysilan

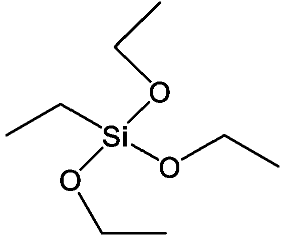

### - n-Hexyltrimethoxysilan

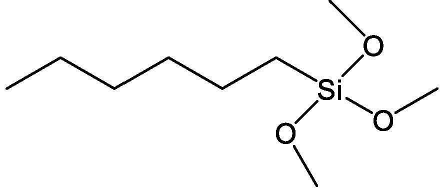

### - n-Hexyltriethoxysilan

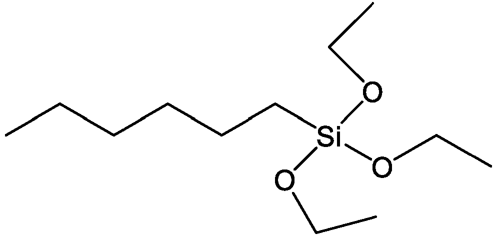

### - n-Octyltrimethoxysilan

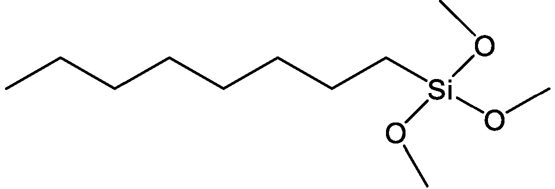

### - n-Octyltriethoxysilan

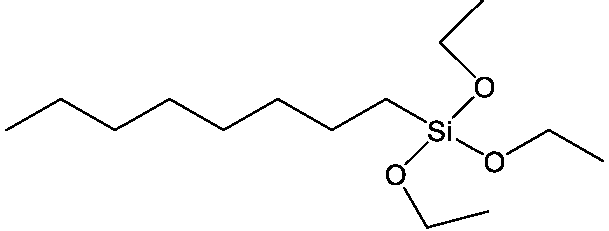

### - n-Dodecyltrimethoxysilan und/oder

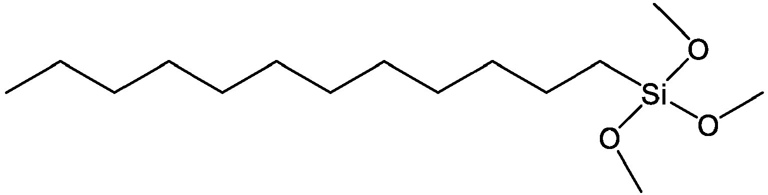

### - n-Dodecyltriethoxysilan.

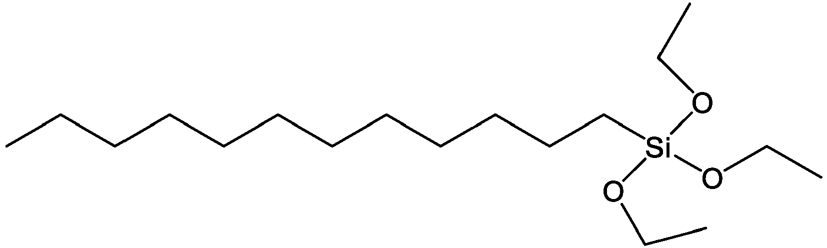

### n-Octadecyltrimethoxysilan und/oder n-Octadecyltriethoxysilan.

In einer weiteren bevorzugten Ausführungsform ist das Verfahren dadurch gekennzeichnet, dass das Mittel (a) mindestens eine organische Siliciumverbindung (a1) der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan
- Dodecyltriethoxysilan
- Octadecyltrimethoxysilan und/oder
- Octadecyltriethoxysilan.

Im Zuge der zu dieser Erfindung führenden Arbeiten hat sich herausgestellt, dass auf dem keratinischen Material auch dann besonders stabile und gleichmäßige Filme erhalten werden konnten, wenn das Mittel (a) zwei strukturell voneinander verschiedene organische Siliciumverbindungen enthält.

Das Mittel (a) enthält mindestens zwei strukturell voneinander verschiedene organische Siliciumverbindungen.

Das Verfahren ist dadurch gekennzeichnet, dass auf dem keratinischen Material ein Mittel (a) angewendet wird, welches mindestens eine organische Siliciumverbindungen der Formel (I) und mindestens eine organische Siliciumverbindungen der Formel (IV) enthält.

In einer explizit ganz besonders bevorzugten Ausführungsform ist das Verfahren dadurch gekennzeichnet, dass auf dem keratinischen Material ein Mittel (a) angewendet wird, welches (a1) mindestens eine organische Siliciumverbindungen der Formel (I) enthält, welche aus der Gruppe bestehend aus (3-Aminopropyl)triethoxysilan und (3-Aminopropyl)trimethoxysilan ausgewäht ist, und zusätzlich mindestens eine organische Siliciumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan ausgewählt ist.

Bei den zuvor beschriebenen organischen Siliciumverbindungen handelt es sich um reaktive Verbindungen. In diesem Zusammenhang hat es sich als bevorzugt herausgestellt, wenn das Mittel (a) - bezogen auf das Gesamtgewicht des Mittels (a) - eine oder mehrere organische Siliciumverbindungen (a1) in einer Gesamtmenge von 0,1 bis 20 Gew.-%, bevorzugt 0,5 bis 15 Gew.- % und besonders bevorzugt 5,0 bis 10 Gew.-% enthält.

In diesem Zusammenhang hat es sich als besonders bevorzugt herausgestellt, wenn das Mittel (a) - bezogen auf das Gesamtgewicht des Mittels (a) - eine oder mehrere organische Siliciumverbindungen (a1) der Formel (I) in einer Gesamtmenge von 0,1 bis 20 Gew.-%, bevorzugt 0,2 bis 15 Gew.-% und besonders bevorzugt 0,2 bis 3 Gew.-% enthält.

Es hat sich weiterhin als besonders bevorzugt herausgestellt, wenn das Mittel (a) - bezogen auf das Gesamtgewicht des Mittels (a) - eine oder mehrere organische Siliciumverbindungen (a1) der Formel (IV) in einer Gesamtmenge von 0,1 bis 20 Gew.-%, bevorzugt 0,5 bis 15 Gew.-% und besonders bevorzugt 2 bis 8 Gew.-% enthält.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem Mittel (a) enthalten sein. Bei Verwendung von Hydrolyse- und/oder Kondensationsprodukte in dem Mittel (a) enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem Mittel (a) enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Ganz besonders gute Ergebnisse konnten erhalten werden, wenn in dem Verfahren organischen Siliciumverbindungen (a1) der Formel (I) und/oder (II) eingesetzt wurden. Da wie bereits zuvor beschrieben bereits bei Spuren von Feuchtigkeit eine Hydrolyse/Kondensation einsetzt, sind auch die Hydrolyse- und/oder Kondensationsprodukte der organischen Siliciumverbindungen (I) und/oder (II) von dieser Ausführungsform mit umfasst.

Besonders resistente Färbungen konnten bei Anwendung eines alkalisch eingestellten Mittels (a) erhalten werden. Bevorzugt enthält das Mittel (a) Wasser und besitzt einen pH-Wert von 7 bis 11,5, bevorzugt von 7,5 bis 11 und besonders bevorzugt von 8 bis 10,5.

In einer weiteren ganz besonders bevorzugten Ausführungsform ist das Verfahren dadurch gekennzeichnet, dass das Mittel (a) einen pH-Wert von 7 bis 11,5, bevorzugt von 7,5 bis 11 und besonders bevorzugt von 8 bis 10,5 besitzt.

### Mittel (b)

Das Mittel (b) ist gekennzeichnet durch die Anwesenheit mindestens einer farbgebende Verbindung (b1) und mindestens ein filmbildendes Polymer (b2). Die farbgebende Verbindung (b1) umfasst mindestens ein Effektpigment, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallpartikelträgerschicht vorgesehen sind.

In den Effektpigmenten werden Interferenzfarben mit hohem Chroma durch Interferenz zwischen dem von der Oberfläche des Substratplättchens reflektierten Licht und dem von den Oberflächen der Metallpartikel reflektierten Licht entwickelt.

Entsprechend können mit Hilfe der "gefärbten" Effektpigmente keratinische Fasern in besonders intensiven und zahlreichen Farbnuancen gefärbt werden.

Das Effektpigment weist ein Substratplättchen auf.

Das Substratplättchen können eine Dicke im Bereich von 10 nm bis 5.000 nm, vorzugsweise im Bereich von 20 nm bis 1.000 nm aufweisen. Es kann jedoch bevorzugt sein, dass die durchschnittliche Dicke höchstens 150 nm, vorzugsweise weniger als 50 nm, mehr bevorzugt weniger als 30 nm, besonders bevorzugt höchstens 25 nm, beispielsweise höchstens 20 nm beträgt. Vorzugsweise weist jedes Substratplättchen eine möglichst einheitliche Dicke auf.

Das Substratplättchen ist vorzugsweise monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb des Substratplättchens Gefügewechsel auftreten können. Das Substratplättchen ist vorzugsweise homogen aufgebaut, d.h. dass innerhalb des Plättchens kein Konzentrationsgradient auftritt. Insbesondere ist das Substratplättchen nicht schichtartig aufgebaut und weist keine darin verteilten Teilchen oder Partikel auf.

Die Größe des Substratplättchens kann auf den jeweiligen Anwendungszweck, beispielsweise dem gewünschten Effekt auf einem keratinischen Material, abgestimmt werden. In der Regel haben die Substratplättchen einen mittleren größten Durchmesser von etwa 2 bis 300 µm, insbesondere etwa 5 bis 100 µm. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Das Substratplättchen kann aus jedem Material, das in Plättchenform gebracht werden kann, aufgebaut sein.

Sie können natürlichen Ursprungs, aber auch synthetisch hergestellt sein. Materialien, aus denen die Substratplättchen aufgebaut sein können, sind beispielsweise Metalle und Metalllegierungen, Metalloxide, vorzugsweise Aluminiumoxid, anorganische Verbindungen und Mineralien wie Glimmer und (Halb)Edelsteine, sowie Kunststoffe. Vorzugsweise sind die Substratplättchen aus einem Metall oder einer Legierung aufgebaut.

Als Metall kommt jedes, für Effektpigmente geeignete Metall in Betracht. Derartige Metalle sind unter anderem Eisen und Stahl, sowie alle luft- und wasserbeständigen (Halb)metalle wie beispielsweise Platin, Zinn, Zink, Chrom, Molybdän und Silicium, sowie deren Legierungen wie Aluminiumbronzen und Messing. Bevorzugte Metalle sind Aluminium, Kupfer, Silber und Gold. Bevorzugte Substratplättchen sind Aluminiumplättchen und Messingplättchen, wobei Substratplättchen aus Aluminium besonders bevorzugt sind.

Substratplättchen aus Aluminium können unter anderem durch Herausstanzen aus Aluminiumfolie oder nach gängigen Mahl- und Verdüsungstechniken hergestellt werden. So sind beispielsweise Aluminiumplättchen aus dem Hallverfahren, einem Nassmahlverfahren, erhältlich.

Andere Metallplättchen, beispielsweise aus Bronze, können in einem Trockenmahlverfahren wie dem Hametagverfahren erhalten werden.

Die Substratplättchen können verschiedene Formen aufweisen. Als Substratplättchen können beispielsweise lamellare oder lentikulare Metallplättchen oder auch sogenannte *vacuum metallized pigments* (VMP) verwendet werden. Lamellare Substratplättchen zeichnen sich durch einen unregelmäßig strukturierten Rand aus und werden aufgrund ihres Erscheinungsbildes auch als "cornflakes" bezeichnet. Lentikulare Substratplättchen weisen einen im Wesentlichen regelmäßigen runden Rand auf und werden aufgrund ihres Erscheinungsbildes auch als "silverdollars" bezeichnet.

Die Substratplättchen aus Metall oder Metalllegierung können passiviert sein, beispielsweise durch Eloxieren (Oxidschicht) oder Chromatieren. Bei einem Substratplättchen aus Aluminium weist dieses beispielsweise eine Passivierungsschicht, umfassend Aluminiumoxid auf.

Das Effektpigment weist mehrere Schichten auf. Insbesondere weist das Effektpigment zumindest eine Metalloxid(hydrat)schicht ii), welche auf der Oberfläche des Substratplättchens vorgesehen ist und eine Metallpartikelträgerschicht iii), welche auf der Oberfläche der Metalloxid(hydrat)schicht vorgesehen ist, auf.

Durch diese Schichten können die Oberflächeneigenschaften und/oder optischen Eigenschaften des Effektpigments verändert sowie die mechanische und chemische Belastbarkeit der Effektpigmente erhöht werden. Es können beispielsweise lediglich die obere und/oder untere Seite des Substratplättchens beschichtet sein, wobei die Seitenflächen ausgespart sind. Vorzugsweise ist die gesamte Oberfläche der gegebenenfalls passivierten Substratplättchen, einschließlich der Seitenflächen, von den weiteren Schichten bedeckt. Die Substratplättchen sind vorzugsweise vollständig von den weiteren Schichten umhüllt.

Auf der Oberfläche des Substratplättchens ist eine Metalloxid(hydrat)schicht ii) vorgesehen. Als Materialien für die Metalloxid(hydrat)schicht ii) eignen sich alle Metalloxid(hydrat)e, die dauerhaft und filmartig auf die Substratplättchen aufgebracht werden können.

Es ist bevorzugt, dass das Metalloxid und/oder Metalloxidhydrat ausgewählt ist aus der Gruppe bestehend aus Silicium(di)oxid, Siliciumoxidhydrat, Aluminiumoxid, Aluminiumoxidhydrat, Boroxid, Germaniumoxid, Manganoxid, Magnesiumoxid, Eisenoxid, Cobaltoxid, Chromoxid, Titandioxid, Vanadiumoxid, Zirkonoxid, Zinnoxid, Zinkoxid und deren Gemischen.

Es ist insbesondere bevorzugt, dass die Metalloxid(hydrat)schicht ii) Siliciumdioxid, insbesondere amorphes Siliciumdioxid, umfasst.

Die Dicke der Metalloxid(hydrat)schicht ii) liegt vorzugsweise im Bereich von 10 bis 500 nm, mehr bevorzugt im Bereich von 10 bis 100 nm.

Während die Metalloxid(hydrat)schicht ii) direkt auf der Oberfläche des Substratplättchens ausgebildet sein kann, ist vorzugsweise eine weitere Schicht zwischen dem Substratplättchen und Metalloxid(hydrat)schicht ii) als Unterschicht angeordnet. Die Unterschicht kann ein Molybdän- und/oder Phosphor-haltiges Oxid, Hydroxid oder Hydrat umfassen. Die Dicke der Unterschicht liegt vorzugsweise im Bereich von 0,5 bis 10 nm. Die Unterschicht kann zusätzlich zu einer Passivierungsschicht oder alternativ zu der Passivierungsschicht auf der Oberfläche des Substratplättches vorhanden sein.

Das Metalloxid(hydrat), insbesondere das Siliciumdioxid, kann beispielsweise nass-chemisch, zum Beispiel nach dem Sol-Gel-Verfahren, aufgebracht werden. Dieses Verfahren umfasst das Dispergieren der unbeschichteten Substratplättchen oder der bereits beschichteten Substratplättchen in einer Lösung eines Metallalkoxids wie Tetraethylorthosilikat oder Aluminiumtriisopropanolat (üblicherweise in einer Lösung von organischem Lösungsmittel oder einer Mischung von organischem Lösungsmittel und Wasser mit mindestens 50 Gew.-% organisches Lösungsmittel wie ein C1 bis C4-Alkohol), und Zugabe einer schwachen Base oder Säure zur Hydrolysierung des Metallalkoxids, wodurch ein Film des Metalloxid(hydrat)s auf der Oberfläche der (beschichteten) Substratplättchen entsteht.

Auf der Oberfläche der Metalloxid(hydrat)schicht ii) ist eine Metallpartikelträgerschicht iii) vorgesehen.

Die Metallpartikelträgerschicht iii) umfasst eine Metallschicht und/oder eine Metalloxidschicht.

Wenn die Metallpartikelträgerschicht iii) eine Metallschicht und einer Metalloxidschicht umfasst, ist die Anordnung dieser Schichten nicht limitiert. Insbesondere kann die Metallpartikelträgerschicht iii) einen Aufbau aufweisen, bei dem die Metallschicht auf der Oberfläche der Metalloxid(hydrat)schicht ii) angeordnet ist und die Metalloxidschicht auf der Oberfläche der Metallschicht angeordnet ist. Die Metallpartikelträgerschicht iii) kann auch einen Aufbau aufweisen, bei dem die Metalloxidschicht auf der Oberfläche der Metalloxid(hydrat)schicht ii) und die Metallschicht auf der Oberfläche der Metalloxidschicht angeordnet ist. Weiterhin kann die Metallpartikelträgerschicht iii) auch zwei oder mehr Metallschichten und/oder zwei oder mehr Metalloxidschichten beinhalten. Die Anordnung der Schichten ist auch hier nicht limitiert.

Es ist bevorzugt, dass die Metallpartikelträgerschicht iii) wenigstens eine Metallschicht umfasst. Es ferner bevorzugt, dass die Metallschicht ein Element ausgewählt aus Zinn (Sn), Palladium (Pd), Platin (Pt) und Gold (Au) aufweist. Insbesondere umfasst die Metallpartikelträgerschicht iii) wenigstens eine Metallschicht, aufweisend ein Element ausgewählt aus Zinn (Sn) und Palladium (Pd).

Während die Metallpartikelträgerschicht iii) direkt auf der Oberfläche der Metalloxid(hydrat)schicht ii) gebildet werden kann, kann noch eine weitere Schicht zwischen der Metalloxid(hydrat)schicht ii) und der Metallpartikelträgerschicht iii) vorhanden sein. Diese weitere Schicht kann beispielsweise ein Oxid oder ein Hydroxid von Aluminium oder Cer umfassen.

Umfasst die Metallpartikelträgerschicht iii) eine Metallschicht kann die Herstellung beispielsweise mittels eines Verfahrens zur hydrolytischen Abscheidung von Metallalkoxid nach einem Sol-Gel-Verfahren oder mittels eines Verfahrens bei dem durch Zugabe von Alkali zu einer Metallsalzlösung, die das Metall zum Bilden der Metallschicht der Metallpartikelträgerschicht iii) enthält, erfolgen.

Erfolgt die Bildung der metallischen Partikel durch stromloses Plattieren, wird die Metallpartikelträgerschicht iii) vorzugsweise durch von Zugabe von Alkali zu einer Metallsalzlösung, die das Metall zum Bilden der Metallschicht der Metallpartikelträgerschicht iii) enthält, gebildet.

Tetraethoxyzinn oder dergleichen kann als Metallalkoxid bei der hydrolytischen Abscheidung von Metallalkoxid verwendet werden. Ammoniakwasser, Ethylendiamin, Monoethanolamin, Diethanolamin, Hydrazin, Harnstoff oder dergleichen kann als hydrolytischer Katalysator eingesetzt werden.

Zinnchlorid, Zinnfluorid oder dergleichen kann als Metallsalz in dem Verfahren, bei dem durch Zugabe von Alkali zu einer Metallsalzlösung, die Metallpartikelträgerschicht iii) gebildet wird, eingesetzt werden. Ammoniakwasser, Natronlauge, Monoethanolamin, Diethanolamin oder dergleichen können als Alkali sowie Wasser, Ethanol, Isopropylalkohol, Methylpropylenglykol, Butylcellosolve oder dergleichen als Lösungsmittel verwendet werden.

Wenn die Metallpartikel durch stromloses Plattieren mit wasserlöslichem Metallsalz gebildet werden, kann die Metallschicht der Metallpartikelträgerschicht iii) durch ein Verfahren gebildet werden, das als Vorbehandlung für die elektrolytische Plattieren eingesetzt wird. Diese Verfahren sind beispielsweise das Katalysator-Beschleunigungsverfahren ("catalyst-accelerator process") oder das Sensitivierungs-Aktivierungsverfahren ("sensitizing-activating process"). Ebenso können nur Teile der Verfahren, beispielsweise das Katalysatorverfahren oder nur das Sensitivierungsverfahren, durchgeführt werden.

Wasserlösliches Metallsalz, das Sn, Pd, Pt oder Au enthält, kann als Metallquelle für die Herstellung der Metallpartikelträgerschicht iii), die bei dem als Vorbehandlung für die stromlose Beschichtung verwendeten Verfahren gebildet wird, eingesetzt werden. Zinnchlorid, Zinnoxalat, Zinnsulfat, Zinnbromid, Zinnacetat, Zinnborfluorid, Zinnfluorid, Natriumstannat, Kaliumstannat, Zinnmesylat, Zinnsulfid, Palladiumchlorid, Palladiumacetat, Palladiumbromid, Palladiumhydroxid, Palladinitrat, Palladiumoxid, Palladiumsulfat, Goldbromid, Goldchlorid, Platinchlorid, Platinchlorid, Platinoxid oder dergleichen sind Beispiele für das wasserlösliche Metallsalz.

Umfasst die Metallpartikelträgerschicht iii) eine Metalloxidschicht, enthält diese vorzugsweise kein Siliciumdioxid. Die Metallpartikelträgerschicht iii) in Form einer Metalloxidschicht enthält vorzugsweise ein Oxid von mindestens einem Element, ausgewählt aus der Gruppe bestehend aus von Mg (Magnesium), Sn (Zinn), Zn (Zink), Co (Kobalt), Ni (Nickel), Fe (Eisen), Zr (Zirkonium), Ti (Titan) und Ce (Cer). Besonders bevorzugt enthält die Metallpartikelträgerschicht iii) in Form einer Metalloxidschicht ein Metalloxid von Sn, Zn, Ti und Ce. Ganz besonders bevorzugt enthält die Metallpartikelträgerschicht iii) in Form einer Metalloxidschicht Zinnoxid.

Die Herstellung der eine Metalloxidschicht einer Metallpartikelträgerschicht iii) kann beispielsweise durch Hydrolyse eines Alkoxids eines Metalls, das das Metall des Metalloxids bildet, in einem Sol-Gel Verfahren, durch Zugabe eines Alkali zu einer Metallsalzlösung eines Metalls, das das Metall des Metalloxids bildet, und durch Inkontaktbringen des beschichteten Substratplättchens mit einer Lösung, in der eine organische Metallverbindung in einem organischen Lösungsmittel gelöst wurde, und anschließender Wärmebehandlung, erfolgen..

Geeignete Metallalkoxide, die in dem Verfahren zum Abscheiden der Metalloxidschicht durch Hydrolyse verwendet werden können, umfassen Tetraethoxyzinn und Tetrabutoxytitan.

Beispiele für ein Metallsalz, das bei dem Verfahren zum Abscheiden der Metalloxidschicht durch Neutralisation mit Alkali verwendet werden kann, sind unter anderem Zinnchlorid, Zinnfluorid, Zinkchlorid, Titanylsulfid, Cernitrat und Ceracetat.

Beispiele für die organische Metallverbindung sind unter anderem Fettsäure-Metallsalze wie Kobaltnaphthenat, Nickelstearat, Zirkoniumstearat und Dibutylzinndilaurat.

Die Wärmebehandlungstemperatur, bei der die organische Metallverbindung zersetzt und oxidiert werden soll, beträgt vorzugsweise 200°C bis 500°C.

Die Metallpartikel können durch stromloses Plattieren auf der Oberfläche der Metallpartikelträgerschicht iii) gebildet werden. Wenn das mit der Metallpartikelträgerschicht iii) versehene Effektpigment in eine Lösung zum stromlose Plattieren getaucht wird, wird aufgrund der katalytischen Aktivität der Metallpartikelträgerschicht iii) ein Reduktionsmittel in der Plattierungslösung auf der Oberfläche der Metallpartikelträgerschicht iii) oxidiert.

Metallionen in der stromlosen Plattierungslösung werden durch die zu diesem Zeitpunkt emittierten Elektronen reduziert, das Metall wird auf der Oberfläche der Metallpartikelträgerschicht abgeschieden und die Metallpartikel werden gebildet.

Die Dicke der Metallpartikelträgerschicht iii) beträgt vorzugsweise bis zu 30 nm und liegt mehr bevorzugt im Bereich von 0,1 bis 10 nm.

Auf der Oberfläche der Metallpartikelträgerschicht iii) sind Metallpartikel iv) aufgebracht. In einer bevorzugten Ausführungsform bedecken die Metallpartikel iv) direkt einen Teil der Metallpartikelträgerschicht iii). In dieser Ausführungsform weist das Effektpigment Bereiche auf, in denen sich keine Metallpartikel befinden, d.h. Bereiche, die nicht mit den Metallpartikeln bedeckt sind.

Die Metallpartikel können wenigstens ein Element umfassen, welches ausgewählt ist aus der Gruppe bestehend aus Aluminium (Al), Titan (Ti), Chrom (Cr), Eisen (Fe), Kobalt (Co), Nickel (Ni), Kupfer (Cu), Zink (Zn), Ruthenium (Ru), Rhodium (Rh), Palladium (Pd), Silber (Ag), Zinn (Sn), Platin (Pt), Gold (Au) und deren Legierungen. Es ist insbesondere bevorzugt, dass die Metallpartikel wenigstens ein Element ausgewählt aus Kupfer (Cu), Nickel (Ni) und Silber (Ag) aufweisen. Es ist äußerst bevorzugt, dass die Metallpartikel Silber (Ag) umfassen.

Der durchschnittliche Partikeldurchmesser der Metallpartikel beträgt vorzugsweise nicht mehr als 50 nm, mehr bevorzugt nicht mehr als 30 nm. Der Abstand zwischen den Metallpartikeln beträgt vorzugsweise nicht mehr als 10 nm.

Als Verfahren zur Bildung der Metallpartikel eignen sich Vakuumverdampfung, Sputtern, Chemical Vapor Deposition (CVD), stromloses Plattieren oder dergleichen. Von diesen Verfahren ist das stromlose Plattieren besonders bevorzugt.

Das Effektpigment kann ferner auf den Metallpartikeln iv) eine Korrosionsinhibierungsschicht, welche einen Korrosionsinhibitor umfasst, aufweisen. Der Korrosionsinhibitor ist vorzugsweise ausgewählt aus der Gruppe der Benzotriazole, Tetrazole, Imidazole, Alkylaminotriazole, Benzothiazole, α- oder β-Dicarbonylverbindungen und Mercaptanen.

Weiterhin kann das Effektpigment auf den Metallpartikeln iv) oder auf der Oberfläche der Korrosionsinhibierungsschicht eine witterungsbeständige Deckschicht, umfassend ein Oxid, ein Hydroxid oder ein Hydrat von Aluminium, Silicium und/oder Cer, aufweisen.

Zusätzlich kann das Effektpigment eine Deckharzschicht oder Deckkunstharzschicht als äußerste Schicht aufweisen.

Solche Effektpigmente sind beispielsweise unter der Bezeichnung Cosmicolor^{®} Celeste von Toyal Europe erhältlich. Geeignete Effektpigmente umfassen beispielsweise Cosmicolor^{®} Celeste Rose Pink, Cosmicolor^{®} Celeste Iris Blue, Cosmicolor^{®} Celeste Morpho Blue, Cosmicolor^{®} CelesteTecla Green, Cosmicolor^{®} Celeste Aqua Green, Cosmicolor^{®} Celeste Meadow Gold, Cosmicolor^{®} Celeste Ivy Orange, Cosmicolor^{®} Celeste Cherry Pink und Cosmicolor^{®} Celeste Maple Red.

Besonders gute Ergebnisse konnten erhalten werden, wenn das Mittel (a) - bezogen auf das Gesamtgewicht des Mittels (a) - ein oder mehrere Effektpigmente in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

Zusätzlich zu dem Effektpigment kann das Mittel (b) weitere farbgebende Verbindungen ausgewählt aus der Gruppe bestehend aus Pigmenten und/oder direktziehenden Farbstoffen umfassen.

Das Mittel (b) ist ferner dadurch gekennzeichnet, dass es mindestens ein filmbildendes Polymer (b2) enthält.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden Polymers (b) nicht mehr als 10⁷ g/mol, bevorzugt nicht mehr als 10⁶ g/mol und besonders bevorzugt nicht mehr als 10⁵ g/mol beträgt.

Unter einem filmbildenden Polymer wird im Sinne der Erfindung ein Polymer verstanden, welches in der Lage ist, auf einem Substrat, beispielsweise auf einem keratinischen Material oder einer keratinischen Faser, einen Film auszubilden. Die Ausbildung eines Films kann beispielsweise durch Betrachtung des mit dem Polymer behandelten keratinischen Materials unter einem Mikroskop nachgewiesen werden.

Die filmbildenden Polymere (b2) im Mittel (b) können hydrophil oder hydrophob sein.

Im Rahmen einer ersten Ausführungsform kann es bevorzugt sein, im Mittel (b) mindestens ein hydrophobes, filmbildendes Polymer einzusetzen.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die Silikon-Polymere, die Polymere des Acrylamid-Typs und die Polyisoprene genannt werden.

Besonders gut geeignete filmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

In einer weiteren bevorzugten Ausführungsform ist ein Mittel (b) dadurch gekennzeichnet, dass es mindestens ein filmbildendes, hydrophobes Polymer (b2) enthält, das ausgewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Zur Lösung der erfindungsgemäßen Aufgabenstellung haben sich insbesondere die filmbildenden hydrophoben Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C₂-C₁₀-Hydroxyalkylgruppe.

Weitere filmbildende hydrophobe Polymere können ausgewählt sein aus den Homo- oder Copolymeren von Isooctyl(meth)acrylat , Isononyl(meth)acrylat , 2-Ethylhexyl(meth)acrylat , Lauryl(meth)acrylat) , Isopentyl(meth)acrylat , n-Butyl(meth)acrylat) , Isobutyl(meth)acrylat , Ethyl(meth)acrylat , Methyl(meth)acrylat , tert-Butyl(meth)acrylat , Stearyl(meth)acrylat , Hydroxyethyl-(meth)acrylat , 2-Hydroxypropyl(methacrylat , 3-Hydroxypropyl(meth)acrylat und/oder Gemischen hiervon.

Weitere filmbildende hydrophobe Polymere können ausgewählt sein aus den Homo- oder Copolymeren von (Meth)acrylamid , N-Alkyl(meth)acrylamiden, insbesondere solchen mit C2-C18 Alkylgruppen, wie beispielsweise N-Ethylacrylamid, N-tert-Butylacrylamid, le N-Octylacrylamid, N-Di(C1-C4)alkyl(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®} 22 (Acrylates/Steareth-20 Methacrylate Copolymer), Aculyn^{®} 28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®} (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyloxazols, des Vinylthiazols, des Vinylpyrimidins oder des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} oder LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copolymere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styren/Butylen, Styren/Isopren, Styren/Butadien. Entsprechende Polymere werden von der BASF unter dem Handelsnamen "Luvitol HSB" kommerziell vertrieben.

Überraschenderweise hat sich herausgestellt, dass dann ganz besonders intensive und waschechte Färbungen erhalten werden konnten, wenn das Mittel (b) mindestens ein filmbildendes Polymer (b2) enthielt, das ausgewählt wurde aus der Gruppe der der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

In einer weiteren bevorzugten Ausführungsform ist ein Verfahren dadurch gekennzeichnet, dass das Mittel (b) mindestens ein filmbildendes Polymer (b2) enthält, das ausgewählt ist aus der Gruppe der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

Im Rahmen einer weiteren Ausführungsform kann es bevorzugt sein, im Mittel (b) mindestens ein hydrophiles, filmbildendes Polymer (b2) einzusetzen.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint makroskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Als filmbildende, hydrophile Polymere können nichtionische, anionische und kationische Polymere eingesetzt werden.

Geeignete filmbildende, hydrophile Polymere können beispielsweise aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummis, der Polysaccharide und/oder der Acrylamid-(Co)Polymere ausgewählt werden.

Weiterhin ist es ganz besonders bevorzugt, als filmbildendes hydrophiles Polymer Polyvinylpyrrolidon (PVP) und/oder ein Vinylpyrrolidon-haltiges Copolymer einzusetzen.

In einer weiteren ganz besonders bevorzugten Ausführungsform ist ein Mittel (b) dadurch gekennzeichnet, dass es mindestens ein filmbildendes, hydrophiles Polymer enthält, das ausgewählt ist aus der Gruppe aus Polyvinylpyrrolidon (PVP) und den Copolymeren des Polyvinylpyrrolidons.

Es ist weiterhin bevorzugt, wenn das Mittel als filmbildendes, hydrophiles Polymer Polyvinylpyrrolidon (PVP) enthält. Überraschenderweise war auch die Waschechtheit der Färbungen, die mit PVPhaltigen Mitteln (b9 erhalten werden konnten, sehr gut.

Besonders gut geeignete Polyvinylpyrrolidone sind beispielsweise unter der Bezeichnung Luviskol^{®} K von der BASF SE erhältlich, insbesondere Luviskol^{®} K 90 oder Luviskol^{®} K 85 der Firma BASF SE.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Der Einsatz von filmbildenden hydrophilen Polymeren (b2) aus der Gruppe der Copolymere des Polyvinylpyrrolidons hat ebenfalls zu besonders guten und waschechten Farbergebnissen geführt.

Als besonders gut geeignete filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionische Polymere.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

In einer weiteren ganz besonders bevorzugten Ausführungsform ist ein Mittel (b) dadurch gekennzeichnet, dass es mindestens ein filmbildendes, hydrophiles Polymer (b2) enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren.

Ein weiteres geeigetes Copolymer des Vinylpyrrolidons ist das unter der INCI Bezeichnung Maltodextrin/VP Copolymer bekannte Polymer.

Weiterhin konnte intensiv gefärbtes keratinischen Material, insbesondere Haare, mit sehr guten Waschechtheiten erhalten werden, wenn als filmbildendes, hydrophiles Polymer ein nichtionisches, filmbildendes, hydrophiles Polymer eingesetzt wurde.

Im Rahmen einer weiteren Ausführungsform kann das Mittel (b) mindestens ein nichtionisches, filmbildendes, hydrophiles Polymer (b2) enthalten.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Es sind die Mittel ganz besonders bevorzugt, die als nichtionisches, filmbildendes, hydrophiles Polymer mindestens ein Polymer enthalten, das ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C1 bis C4)-Alkylamino-(C2 bis C4)-alkylacrylamid.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®} CC 10 von der Firma ISP verkauft wird.

Ein kationisches Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-Wasser-Gemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Als geeignete anionische filmbildende, hydrophile Polymere können zum Beispiel Acrylsäure-Polymere eingesetzt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Beispiele für geeignete filmbildende, hydrophile Polymere aus der Gruppe der natürlichen Gums sind Xanthan Gum, Gellan Gum, Carob Gum.

Beispiele für geeignete filmbildende, hydrophile Polymere aus der Gruppe der Polysaccharide sind Hydroxyethylcellulose, Hydroxypropylcellulose, Ethylcellulose und Carboxymethyl-Cellulose.

Geeignete filmbildende, hydrophile Polymere aus der Gruppe der Acrylamde sind beispielsweise Polymere, welche ausgehend von Monomeren der (Meth)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure.

Bevorzugte Polymere der Poly(meth)arylamido-C1-C4-alkyl-sulfonsäuren sind vernetzt und zu mindestens 90 % neutralisiert. Diese Polymere können vernetzt oder auch unvernetzt sein.

Vernetzte und ganz oder teilweise neutralisierte Polymere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres bevorzugtes Polymer dieses Typs ist das der Firma Clariant unter dem Handelsnamen Hostacerin AMPS vertriebene vernetzte Poly-2-acrylamido-2methyl-propanesulphonsäure-Polymer, das teilweise mit Ammoniak neutralisiert ist.

In einer weiteren explizit ganz besonders bevorzugten Ausführungsform ist ein Verfahren dadurch gekennzeichnet, dass das Mittel (b) mindestens ein anionisches, filmbildendes, Polymer (b2) enthält.

In diesem Zusammenhang konnten die besten Ergebnisse erhalten werden, wenn das Mittel (b) mindestens ein filmbildendes Polymer (b2) enthält, das mindestens eine Struktureinheit der Formel (P-I) und mindestens eine Struktureinheit der Formel (P-II) umfasst 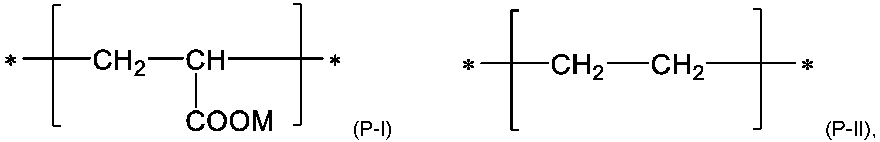 wobei
M für ein Wasserstoffatom oder für Ammonium (NH₄), Natrium, Kalium, ½ Magnesium oder ½ Calcium steht.

In einer weiteren bevorzugten Ausführungsform ist ein Verfahren dadurch gekennzeichnet, dass das Mittel (b) mindestens ein filmbildendes Polymer (b2) enthält, das mindestens eine Struktureinheit der Formel (P-I) und mindestens eine Struktureinheit der Formel (P-II) umfasst 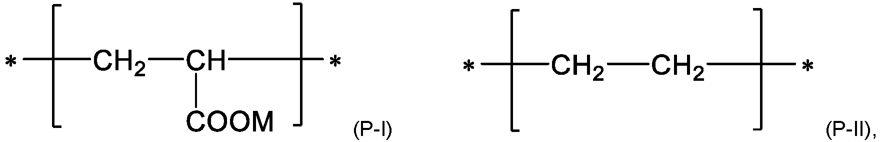 wobei
M für ein Wasserstoffatom oder für Ammonium (NH₄), Natrium, Kalium, ½ Magnesium oder ½ Calcium steht.

Wenn M für ein Wasserstoffatom steht, basiert die Struktureinheit der Formel (P-I) auf einer Acrylsäure-Einheit.

Wenn M für ein Ammonium-Gegenion steht, basiert die Struktureinheit der Formel (P-I) auf dem Ammoniumsalz der Acrylsäure.

Wenn M für ein Natrium-Gegenion steht, basiert die Struktureinheit der Formel (P-I) auf dem Natriumsalz der Acrylsäure.

Wenn M für ein Kalium-Gegenion steht, basiert die Struktureinheit der Formel (P-I) auf dem Kaliumsalz der Acrylsäure.

Wenn M für ein halbes Äquivalent eines Magnesium-Gegenions steht, basiert die Struktureinheit der Formel (P-I) auf dem Magnesiumsalz der Acrylsäure.

Wenn M für ein halbes Äquivalent eines Calcium-Gegenions steht, basiert die Struktureinheit der Formel (P-I) auf dem Calciumsalz der Acrylsäure.

Das oder die filmbildenden Polymere (b2) werden bevorzugt in bestimmten Mengenbereichen in dem Mittel (b) eingesetzt. In diesem Zusammenhang hat es sich zur Lösung der erfindungsgemäßen Aufgabenstellung als besonders bevorzugt erwiesen, wenn das Mittel (b) - bezogen auf das Gesamtgewicht des Mittels (b) - ein oder mehrere filmbildende Polymere (b2) in einer Gesamtmenge von 0,1 bis 18 Gew.-%, bevorzugt von 1 bis 16 Gew.-%, weiter bevorzugt von 5 bis 14,5 Gew.-% und ganz besonders bevorzugt von 8 bis 12 Gew.-% enthält.

In einer weiteren bevorzugten Ausführungsform ist ein Verfahren dadurch gekennzeichnet, dass das Mittel (b) - bezogen auf das Gesamtgewicht des Mittels (b) - ein oder mehrere filmbildende Polymere (b2) in einer Gesamtmenge von 0,1 bis 18 Gew.-%, bevorzugt von 1 bis 16 Gew.-%, weiter bevorzugt von 5 bis 14,5 Gew.-% und ganz besonders bevorzugt von 8 bis 12 Gew.-% enthält.

### Weitere Inhaltsstoffe in den Mitteln (a) und (b)

Die zuvor beschriebenen Mittel (a) und (b) können ferner auch noch ein oder mehrere optionale Inhaltsstoffe enthalten.

Es ist insbesondere bevorzugt, dass das in dem Färbeverfahren angewendete Mittel (a) mindestens eine farbgebende Verbindung (a2) ausgewählt aus der Gruppe der Pigmente und/oder der direktziehenden Farbstoffe umfasst.

Der Einsatz von Pigmenten hat sich in diesem Zusammenhang als ganz besonders bevorzugt herausgestellt.

In einer weiteren ganz besonders bevorzugten Ausführungsform ist ein Verfahren dadurch gekennzeichnet, dass das Mittel (a) mindestens eine farbgebende Verbindung (a2) aus der Gruppe der Pigmente enthält.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Pigmente können anorganischen und/oder organischen Ursprungs sein.

In einer bevorzugten Ausführungsform ist ein Verfahren dadurch gekennzeichnet, dass das Mittel (a) mindestens eine farbgebende Verbindung (a2) aus der Gruppe der anorganischen und/oder organischen Pigmente enthält.

Bevorzugte Pigmente sind ausgewählt aus synthetischen oder natürlichen anorganischen Pigmenten. Anorganische Pigmente natürlichen Ursprungs können beispielsweise aus Kreide, Ocker, Umbra, Grünerde, gebranntem Terra di Siena oder Graphit hergestellt werden. Weiterhin können als anorganische Pigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Pigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Ebenfalls besonders bevorzugte Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

Ebenfalls bevorzugte Pigmente auf Glimmerbasis sind mit Metalloxid beschichtete synthetisch hergestellte Glimmerplättchen, insbesondere basierend auf synthetischem Fluorphlogopit (INCI: Synthetic Fluorphlogopite). Die synthetischen Fluorphlogopitplättchen sind beispielsweise mit Zinnoxid, Eisenoxid(en) und/oder Titandioxid beschichtet. Die Metalloxidschichten können ferner Pigmente wie Eisen(III)-hexacyanidoferrat(II/III) oder Karminrot aufweisen. Solche Glimmerpigmente sind beispielsweise unter der Bezeichnung SYNCRYSTAL von Eckart erhältlich.

Entsprechend ist ein bevorzugtes Verfahren dadurch gekennzeichnet, dass das Mittel (a) mindestens eine farbgebende Verbindung (a2) aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist das Verfahren dadurch gekennzeichnet, dass das Mittel (a) mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Weitere geeignete Pigmente basieren auf Metalloxid-beschichteten plättchenförmigen Borosilikaten. Diese sind beispielsweise mit Zinnoxid, Eisenoxid(en), Siliciumdioxid und/oder Titandioxid beschichtet. Solche Borosilikat-basierten Pigmente sind beispielsweise unter der Bezeichnung MIRAGE von Eckart oder Reflecks von BASF SE erhältlich.

Beispiele für besonders geeignete Pigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors, Flamenco^{®}, Cellini^{®}, Cloisonne^{®}, Duocrome^{®}, Gemtone^{®}, Timica^{®}, MultiReflections, Chione von der Firma BASF SE und Sunshine^{®} von der Firma Sunstar erhältlich.
Ganz besonders bevorzugte Pigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)
Colorona SynCopper, Merck, Synthetic Fluorphlogopite (and) Iron Oxides
Colorona SynBronze, Merck, Synthetic Fluorphlogopite (and) Iron Oxides
Weiterhin besonders bevorzugte Pigmente mit der Handelsbezeichnung Xirona^{®} sind beispielsweise: Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide Xirona Le Rouge, Merck, Iron Oxides (and) Silica
Zudem sind besonders bevorzugte Pigmente mit der Handelsbezeichnung Unipure^{®} beispielsweise:
   Unipure Red LC 381 EM, Sensient CI 77491 (Iron Oxides), Silica
   Unipure Black LC 989 EM, Sensient, CI 77499 (Iron Oxides), Silica
   Unipure Yellow LC 182 EM, Sensient, CI 77492 (Iron Oxides), Silica
   Ebenfalls besonders bevorzugte Pigmente mit der Handelsbezeichnung Flamenco^{®} sind beispielsweise:
      Flamenco^{®} Summit Turquoise T30D, BASF, Titanium Dioxide (and) Mica
      Flamenco^{®} Super Violet 530Z, BASF, Mica (and) Titanium Dioxide

Im Rahmen einer weiteren Ausführungsform kann das in dem Verfahren eingesetzte Mittel (a) auch ein oder mehrere farbgebende Verbindungen (a2) aus der Gruppe der organischen Pigmente enthalten.

Bei den organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist das Verfahren dadurch gekennzeichnet, dass das Mittel (a) mindestens eine farbgebende Verbindungen (a2) aus der Gruppe der organischen Pigmente enthält, die ausgewählt ist aus der Gruppe bestehend aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Ebenfalls geeignete farbgebende Verbindungen (a2) aus der Gruppe der Pigmente sind anorganische und/oder organische Pigmente, die mit einem Polymer modifiziert wurden. Durch die Polymermodifikation kann beispielsweise die Affinität der Pigmente zu dem von der organischen Siliciumverbindung (a1) auf dem keratinischen Material gebildetem Film erhöht werden.

Es können auch andere Effektpigmente, wie beispielsweise Metallglanzpigmente, als farbgebende Verbindung (a2) eingesetzt werden.

Die Effektpigmente können beispielsweise Pigmente auf Basis eines lamellaren Substratplättchens, Pigmente auf Basis von lentikularen Substratplättchen, Pigmente auf Basis von Substratplättchen, die "vacuum metallized pigments" (VMP) umfassen, enthalten.

Geeignete Effektpigmente umfassen beispielsweise die Pigmente Alegrace^{®} Marvelous, Alegrace^{©} Gorgeous oder Alegrace^{®} Aurous von Schlenk Metallic Pigments.

Ebenfalls geeignete Effektpigmente sind die Aluminium-basierten Pigmente der Reihe SILVERDREAM sowie die auf Aluminium oder auf kupfer-/ zinkhaltigen Metalllegierungen basierenden Pigmente der Reihe VISIONAIRE von Eckart.

Weitere geeignete Effektpigmente basieren auf Metalloxid-beschichteten plättchenförmigen Borosilikaten. Diese sind beispielsweise mit Zinnoxid, Eisenoxid(en), Siliciumdioxid und/oder Titandioxid beschichtet. Solche Borosilikat-basierten Pigmente sind beispielsweise unter der Bezeichnung MIRAGE von Eckart oder Reflecks von BASF SE erhältlich.

Aufgrund ihrer ausgezeichneten Licht- und Temperaturbeständigkeit ist die Verwendung der zuvor genannten Pigmente in dem Mittel (a) besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1 bis 50 µm, vorzugsweise von 5 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

In einer weiteren bevorzugten Ausführungsform ist das Verfahren dadurch gekennzeichnet, dass das Mittel (a) - bezogen auf das Gesamtgewicht des Mittels (a) - ein oder mehrere farbgebende

Verbindung(en) (a2) in Form von Pigmenten in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

Als farbgebende Verbindung(en) (a2) können die in dem Verfahren angewendeten Mittel (a) auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1 g/L.

Direktziehende Farbstoffe können in anionische, kationische und nichtionische direktziehende Farbstoffe unterteilt werden.

In einer weiteren bevorzugten Ausführungsform ist das Verfahren dadurch gekennzeichnet, dass das Mittel (a) ferner als farbgebende Verbindung (a2) mindestens einen anionischen, kationischen und/oder nichtionischen direktziehenden Farbstoff enthält.

In einer weiteren bevorzugten Ausführungsform ist das Verfahren dadurch gekennzeichnet, dass das Mittel (a) ferner mindestens eine farbgebende Verbindung (a2) aus der Gruppe der anionischen, nichtionischen, und/oder kationischen direktziehenden Farbstoffe enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeignete nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Im Zuge der zu dieser Erfindung führenden Arbeiten hat sich herausgestellt, dass insbesondere mit Mitteln (a), die mindestens einen anionischen direktziehenden Farbstoff enthalten, Färbungen mit besonders hoher Farbintensität erzeugt werden können.

In einer explizit ganz besonders bevorzugten Ausführungsform ist das Verfahren daher dadurch gekennzeichnet, dass das Mittel (a) ferner mindestens einen anionischen direktziehenden Farbstoff als farbgebende Verbindung (a2) enthält.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Die Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer Ausführungsform bevorzugt ist damit ein Verfahren zum Färben von keratinischem Material, welches dadurch gekennzeichnet ist, dass das Mittel (a) ferner mindestens einen anionischen direktziehenden Farbstoff als farbgebende Verbindung (a2) enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe (-SO₃H) eine Natriumsulfonatgruppe (-SO₃Na) und/oder eine Kaliumsulfonatgruppe (-SO₃K) besitzen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, Cl 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt.

Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intensität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriumsalze von Mono- und Disulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatriumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 Gew.-%.

Acid Red 33 ist das Dinatriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Ein ganz besonders bevorzugtes Verfahren ist daher dadurch gekennzeichnet, dass das Mittel (a) mindestens eine erste farbgebende Verbindung (a2) aus der Gruppe der anionischen direktziehenden Farbstoffe enthält, die ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe, insbesondere die anionischen direktziehenden Farbstoffe, können je nach erwünschter Farbintensität in verschiedenen Mengen im Mittel (a) eingesetzt werden. Besonders gute Ergebnisse konnten erhalten werden, wenn das Mittel (a) - bezogen auf sein Gesamtgewicht - ein oder mehrere direktziehende Farbstoffe (a2) in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

In einer weiteren bevorzugten Ausführungsform ist das Verfahren dadurch gekennzeichnet, dass das Mittel (a) - bezogen auf das Gesamtgewicht des Mittels (a) - ferner ein oder mehrere direktziehende Farbstoffe als farbgebende Verbindung (a2) in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

Die Mittel können zusätzlich ein oder mehrere Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -COO⁽⁻⁾ - oder-SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ -Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ -Acylsarcosin.

Die Mittel können auch zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Die anionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Zur Einstellung des gewünschten pH-Wertes können die Mittel (a) und (b) auch mindestens ein Alkalisierungsmittel und/oder Acidifizierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können die Mittel (a) und (b) beispielsweise Ammoniak, Alkanolamine und/oder basische Aminosäuren enthalten.

Die in den Mitteln einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass ein Mittel (a) und/oder (b) als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus können die Mittel (a) und/oder (b) weitere Alkalisierungsmittel, insbesondere anorganische Alkalisierungsmittel enthalten. Einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Dem Fachmann geläufige Acidifizierungsmittel sind beispielsweise organische Säuren, wie beispielsweise Citronensäure, Essigsäure, Maleinsäure, Milchsäure, Äpfelsäure oder Weinsäure, sowie verdünnte Mineralsäuren, wie beispielsweise Salzsäure, Schwefelsäure oder Phosphorsäure.

Das Mittel (a) und/oder das Mittel (b) können ferner ein Mattierungsmittel enthalten. Geeignete Mattierungsmittel umfassen beispielsweise (modifizierte) Stärken, Wachse, Talkum und/oder (modifizierte) Kieselsäuren. Die Menge an Mattierungsmittel liegt bevorzugt zwischen 0,1 und 10 Gew.-% bezogen auf die Gesamtmenge an Mittel (a) oder Mittel (b). Bevorzugt enthält Mittel (b) ein Mattierungsmittel.

Die Mittel (a) und/oder (b) können ferner auch noch weitere Wirk-, Hilfs- und Zusatzstoffe enthalten, wie beispielsweise Lösungsmittel, Fettbestandteile wie beispielsweise der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Polymere; Strukturanten wie Glucose oder Natriumchlorid, haarkonditionierende Verbindungen wie Phospholipide, beispielsweise Lecithin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den Mitteln (a) und/oder (b) bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Verfahren zum Färben von keratinischen Materialien

Im Rahmen des Verfahrens werden die Mittel (a) und (b) auf die keratinischen Materialien, insbesondere auf die menschlichen Haare, appliziert. Damit sind die Mittel (a) und (b) die anwendungsbereiten Mittel. Die Mittel (a) und (b) sind voneinander verschieden.

Die Mittel (a) und (b) können prinzipiell gleichzeitig oder sukzessive angewendet werden, wobei die sukzessive Anwendung bevorzugt ist.

Die besten Ergebnisse konnten erhalten werden, wenn das Mittel (a) als Vorbehandlungsmittel auf die keratinischen Materialien gegeben wurde und danach das Mittel (b) als Färbemittel angewendet wurde.

Ganz besonders bevorzugt ist daher ein Verfahren zum Färben von keratinischem Material, insbesondere menschlichen Haaren, umfassend die folgenden Schritte in der angegebenen Reihenfolge:
- in einem ersten Schritt Anwendung eines Mittels (a) auf dem keratinischem Material, wobei das Mittel (a) mindestens zwei organische Siliciumverbindungen (a1) enthält, und
- in einem zweiten Schritt Anwendung eines Mittels (b) auf dem keratinischen Material, wobei das Mittel (b) enthält:
   (b1) mindestens eine farbgebende Verbindung, umfassend mindestens ein Effektpigment, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallschicht vorgesehen sind, und
   (b2) mindestens ein filmbildendes Polymer.

Um dem gefärbten keratinischen Material über einen längeren Zeitraum eine hohe Auswaschresistenz zu verleihen, werden die Mittel (a) und (b) zudem besonders bevorzugt innerhalb ein und desselben Färbeverfahrens angewendet, was bedeutet, dass zwischen der Anwendung der Mittel (a) und (b) ein Zeitraum von maximal einigen Stunden liegt.

Im Rahmen einer weiteren bevorzugten Ausführungsform ist das Verfahren dadurch gekennzeichnet, das zunächst das Mittel (a) angewendet wird und danach das Mittel (b) angewendet wird, wobei der Zeitraum zwischen der Anwendung der Mittel (a) und (b) bei maximal 24 Stunden, bevorzugt bei maximal 12 Stunden und besonders bevorzugt bei maximal 6 Stunden liegt.

Im Rahmen des Verfahrens werden die keratinischen Materialien, insbesondere die menschlichen Haare, zunächst mit Mittel (a) behandelt. Im Anschluss daran wird das eigentliche Färbemittel (b) - welches die farbgebenden Verbindungen enthält - auf die keratinischen Materialien gegeben.

Kennzeichnend für das Vorbehandlungsmittel (a) ist sein Gehalt an mindestens zwei reaktiven organische Siliciumverbindungen. Die reaktiven organischen Siliciumverbindungen (a) funktionalisieren die Haaroberfläche, sobald sie mit dieser in Kontakt kommen. Auf diesem Wege wird ein erster Film ausgebildet. Im zweiten Schritt des Verfahrens wird nun ein Färbemittel (b) auf die Haare aufgetragen. Während der Anwendung des Färbemittels (b) gehen die farbgebenden Verbindungen eine Wechselwirkung mit dem von den siliciumorganischen Verbindungen gebildeten Film ein und werden auf diese Weise an die keratinischen Materialien gebunden.

Im Rahmen einer weiteren Ausführungsform ganz besonders bevorzugt ist ein Verfahren, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

Unter dem Ausspülen des keratinischen Materials mit Wasser in den Schritten (3) und (6) des Verfahrens wird erfindungsgemäß verstanden, dass für den Ausspülvorgang nur Wasser verwendet wird, ohne dass noch weitere, von den Mitteln (a) und (b) verschiedene Mittel zur Anwendung kämen.

In einem Schritt (1) wird zunächst das Mittel (a) auf die keratinischen Materialien, insbesondere die menschlichen Haare, appliziert.

Nach dem Auftragen wird das Mittel (a) auf die keratinischen Materialien einwirken gelassen. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf die keratinischen Materialien, insbesondere auf menschliche Haare, haben sich als besonders vorteilhaft erwiesen.

Im Rahmen einer bevorzugten Ausführungsform des Verfahrens kann das Mittel (a) nun von den keratinischen Materialien ausgespült werden, bevor das Mittel (b) im nachfolgenden Schritt auf die Haare appliziert wird.

Färbungen mit ebenfalls guten Waschechtheiten wurden erhalten, wenn das Mittel (b) auf die keratinischen Materialien appliziert wurde, die noch mit dem Mittel (a) beaufschlagt waren.

In Schritt (4) wird nun das Mittel (b) auf die keratinischen Materialien appliziert. Nach dem Auftragen wird nun das Mittel (b) auf die Haare einwirken gelassen.

Das Verfahren erlaubt selbst bei kurzer Einwirkzeit des Mittels (b) die Erzeugung von Färbungen mit besonders guter Intensität und Waschechtheit. Einwirkzeiten von 10 Sekunden bis 10 Minuten, bevorzugt von 20 Sekunden bis 5 Minuten und ganz besonders bevorzugt von 30 Sekunden bis 3 Minuten auf den keratinischen Materialien, insbesondere auf menschlichen Haaren, haben sich als besonders vorteilhaft erwiesen.

In Schritt (6) wird nun das Mittel (b) (sowie ggf. noch vorhandenes Mittel (a)) mit Wasser aus dem keratinischen Material ausgespült.

### Mehrkomponenten-Verpackungseinheit (Kit-of-parts)

Im Rahmen des Verfahrens werden die Mittel (a) und (b) auf den keratinischen Materialien angewendet, d.h. bei den zwei Mitteln (a) und (b) handelt es sich jeweils um die anwendungsbereiten Mittel.

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Ein zweiter Gegenstand der vorliegenden Erfindung ist daher eine Mehrkomponenten-Verpackungseinheit (Kit-of-parts) zum Färben von keratinischem Material, umfassend getrennt voneinander konfektioniert
einen ersten Container mit einem Mittel (a), wobei das Mittel (a) mindestens eine organische Siliciumverbindung (a1) enthält, umfassend mindestens eine organische Siliciumverbindungen der Formel (I) und mindestens eine organische Siliciumverbindungen der Formel (IV) enthält, wobei in der organischen Siliciumverbindung der Formel (I)

   R₁R₂N-L-Si(OR₃)ₐ(R₄)_{b} (I),

   - R₁, R₂ unabhängig voneinander für ein Wasserstoffatom oder eine C₁-C₆-Alkylgruppe stehen,
   - L für eine lineare oder verzweigte, zweiwertge C₁-C₂₀-Alkylengruppe steht,
      - R₃ für ein Wasserstoffatom oder für eine C₁-C₆-Alkylgruppe steht,
   - R₄ für eine C₁-C₆-Alkylgruppe steht,
   - a, für eine ganze Zahl von 1 bis 3 steht, und
   - b für die ganze Zahl 3 - a steht, und
wobei in der organischen Siliciumverbindung der Formel (IV)

   R₉Si(OR₁₀)ₖ(R₁₁)ₘ (IV),

   - R₉ für eine C₁-C₁₈-Alkylgruppe steht,
   - R₁₀ für ein Wasserstoffatom oder eine C₁-C₆-Alkylgruppe steht,
   - R₁₁ für eine C₁-C₆-Alkylgruppe steht
   - k für eine ganze Zahl von 1 bis 3 steht, und
   - m für die ganze Zahl 3 - k steht, und

- einen zweiten Container mit einem Mittel (b), wobei das Mittel (b) enthält:
   (b1) mindestens eine farbgebende Verbindung, umfassend mindestens ein Effektpigment, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallschicht vorgesehen sind, und
   (b2) mindestens ein filmbildendes Polymer.

Die im Mittel (a) des Kits enthaltenen organische Siliciumverbindungen entsprechen den organischen Siliciumverbindungen, die auch im Mittel (a) des zuvor beschriebenen Verfahrens eingesetzt wurden.

Die im Mittel (b) des Kits enthaltenen farbgebenden Verbindungen aus der Gruppe der Effektpigmente, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallschicht vorgesehen sind, entsprechen den farbgebenden Verbindungen aus der Gruppe der Effektpigmente, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallschicht vorgesehen sind, die auch im Mittel (b) des zuvor beschriebenen Verfahrens eingesetzt wurden.

Das Mittel (a) enthält mit der oder den organischen Siliciumverbindungen eine Klasse von reaktiven Verbindungen, die wie zuvor beschriebenen in Anwesenheit von Wasser eine Hydrolyse und/oder Oligomerisierung und/oder Polymerisierung eingehen können. Infolge ihrer hohen Reaktivität bilden diese organischen Siliciumverbindungen auf dem keratinischen Material einen Film aus.

Zur Vermeidung einer vorzeitigen Hydrolyse, Oligomerisierung und/oder Polymerisierung kann es bevorzugt sein, dass anwendungsbereite Mittel (a) erst kurz vor der Anwendung herzustellen.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') mindestens eine organische Siliciumverbindung enthält, umfassend mindestens eine organische Siliciumverbindungen der Formel (I) und mindestens eine organische Silicium-verbindungen der Formel (IV) enthält, wobei in der organischen Siliciumverbindung der Formel (I)
   - R1R2N-L-Si(OR3)a(R4)b (I),
   - R1, R2 unabhängig voneinander für ein Wasserstoffatom oder eine C1-C6-Alkylgruppe stehen,
   - L für eine lineare oder verzweigte, zweiwertge C1-C20-Alkylengruppe steht,
   - R3 für ein Wasserstoffatom oder für eine C1-C6-Alkylgruppe steht,
   - R4 für eine C1-C6-Alkylgruppe steht,
   - a, für eine ganze Zahl von 1 bis 3 steht, und
   - b für die ganze Zahl 3 - a steht, und
   - wobei in der organischen Siliciumverbindung der Formel (IV)
   - R9Si(OR10)k(R11)m (IV),
   - R9 für eine C1-C18-Alkylgruppe steht,
   - R10 für ein Wasserstoffatom oder eine C1-C6-Alkylgruppe steht,
   - R11 für eine C1-C6-Alkylgruppe steht
   - k für eine ganze Zahl von 1 bis 3 steht, und
   - m für die ganze Zahl 3 - k steht
- einen zweiten Container mit einem Mittel (a"), wobei das Mittel (a") Wasser enthält, und
- einen dritten Container mit einem Mittel (b), wobei das Mittel (b) enthält:
   (b1) mindestens eine farbgebende Verbindung, umfassend mindestens ein Effektpigment, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallpartikelträgerschicht vorgesehen sind, und
   (b2) mindestens ein filmbildendes Polymer.

Um eine möglichst lagerstabile Formulierung bereitstellen zu können, wird das Mittel (a') selbst bevorzugt wasserarm oder wasserfrei konfektioniert.

In einer bevorzugten Ausführungsform ist eine Mehrkomponenten-Verpackungseinheit (Kit-of-parts) dadurch gekennzeichnet, dass das Mittel (a') - bezogen auf das Gesamtgewicht des Mittels (a') - einen Wassergehalt von weniger als 10 Gew.-%, bevorzugt von weniger als 5 Gew.-%, weiter bevorzugt von weniger als 1 Gew.-%, noch weiter bevorzugt von weniger als 0,1 Gew.-% und ganz besonders bevorzugt von weniger als 0,01 Gew.-% besitzt.

Das Mittel (a") enthält Wasser. In einer bevorzugten Ausführungsform ist eine Mehrkomponenten-Verpackungseinheit (Kit-of-parts) dadurch gekennzeichnet, dass das Mittel (a") - bezogen auf das Gesamtgewicht des Mittels (a") - einen Wassergehalt von 15 bis 100 Gew.-%, bevorzugt von 35 bis 100 Gew.-%, weiter bevorzugt von 55 bis 100 Gew.-%, noch weiter bevorzugt von 65 bis 100 Gew.-% und ganz besonders bevorzugt von 75 bis 100 Gew.-% besitzt.

Innerhalb dieser Ausführungsform wird das anwendungsbereite Mittel (a) nun durch Vermischen der Mittel (a') und (a") hergestellt.

Beispielsweise kann der Anwender das Mittel (a'), welches die organische Siliciumverbindung(en) enthält, zunächst mit dem wasserhaltigen Mittel (a") verrühren oder verschütteln. Diese Mischung aus (a') und (a") kann der Anwender nun - entweder direkt nach ihrer Herstellung oder nach einer kurzen Reaktionszeit von 10 Sekunden bis 20 Minuten - auf die keratinischen Materialien applizieren. Im Anschluss daran kann der Anwender wie zuvor beschrieben das Mittel (b) anwenden.

In dieser Ausführungsform der Mehrkomponenten-Verpackungseinheit kann es bevorzugt sein, dass das Mittel (a") ferner mindestens eine farbgebende Verbindung (a2) enthält. Diese ist vorzugsweise ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

Alternativ kann Mehrkomponenten-Verpackungseinheit ferner ein Mittel (a‴) umfassen, das mindestens eine farbgebende Verbindung (a2) enthält. Vorzugsweise enthält das Mittel (a‴) ferner ein Silikonpolymer.

Innerhalb dieser Ausführungsform wird das anwendungsbereite Mittel (a) nun durch Vermischen der Mittel (a'), (a") und (a‴) hergestellt.

Betreffend die weiteren bevorzugten Ausführungsformen der Mehrkomponenten-Verpackungseinheit gilt *mutatis mutantis* das zum Verfahren Gesagte.

Beispiele

### 1. Formulierungen

Es wurden die folgenden Formulierungen hergestellt (sofern nichts anderes angegeben ist, sind alle Angaben in Gew.-%)

| Vorbehandlungsmittel, Mittel (a) | |
|---|---|
| (3-Aminopropyl)triethoxysilan | 2,0 |
| Methyltrimethoxysilan | 7,0 |
| Ammoniak/Citronensäure | ad pH 9,5 |
| Wasser | ad 100 |

Die Silane wurden mit einem Teil des Wassers vermischt, diese Mischung wurde für 30 Minuten stehen gelassen. Dann wurde der pH-Wert durch Zugabe von Citronensäure/Ammoniak auf den gewünschten Wert eingestellt. Anschließend wurde mit Wasser auf 100 g aufgefüllt.

| Färbemittel, Mittel (b) | |
|---|---|
| Cosmicolor^{®} Celeste Cherry Pink | 1 |
| PVP K 30 (Ashland, ISP, Polyvinylpyrrolidone) | 4,5 |
| Dermacryl 79 (Akzo Nobel, Acrylates/Octylacrylamide Copolymer, CAS-Nr. 129702-02-9) | 4,5 |
| Ammoniak (25 %ige wässrige Lösung) | ad pH 10 |
| Wasser | ad 100 |

### 2. Anwendung

Jeweils eine Haarsträhne (Kerling, Euronaturhaar weiß) wurde in das Mittel (a) getaucht und für 1 Minuten darin belassen. Danach wurde überflüssiges Mittel (a) von jeder Haarsträhne gestreift. Jede Haarsträhne wurde mit Wasser kurz ausgewaschen. Überschüssiges Wasser wurde von jeder Haarsträhne gestreift.

Im Anschluss daran wurden die Haarsträhnen jeweils in das Mittel (b) getaucht und für 1 Minute darin belassen. Danach wurde überflüssiges Mittel (b) von jeder Haarsträhne gestreift. Jede Haarsträhne wurde mit Wasser kurz ausgewaschen. Überschüssiges Wasser wurde von jeder Haarsträhne gestreift.

Im Anschluss daran wurden die Strähnen visuell bewertet

## Patentansprüche

1. Verfahren zum Färben von keratinischem Material, insbesondere menschlichen Haaren, umfassend die folgenden Schritte:
- Anwendung eines Mittels (a) auf dem keratinischen Material, wobei das Mittel (a) enthält: mindestens eine organische Siliciumverbindungen der Formel (I) und mindestens eine organische Siliciumverbindungen der Formel (IV), wobei in der organischen Siliciumverbindung der Formel (I)
R₁R₂N-L-Si(OR₃)ₐ(R₄)_{b} (I),
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder für eine C₁-C₆-Alkylgruppe steht,
- R₄ für eine C₁-C₆-Alkylgruppe steht,
- a, für eine ganze Zahl von 1 bis 3 steht, und
- b für die ganze Zahl 3 - a steht, und
wobei in der organischen Siliciumverbindung der Formel (IV)
R₉Si(OR₁₀)ₖ(R₁₁)ₘ (IV),
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht, und
- Anwendung eines Mittels (b) auf dem keratinischen Material, wobei das Mittel (b) enthält:
(b1) mindestens eine farbgebende Verbindung, umfassend mindestens ein Effektpigment, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallpartikelträgerschicht vorgesehen sind, und
(b2) mindestens ein filmbildendes Polymer.

2. Verfahren nach Anspruch 1, **dadurch gekennzeichnet, dass** das Mittel (a) ferner mindestens eine organische Siliciumverbindung der Formel (II) enthält
(R₅O)_{c}(R₆)_{d}Si-(A)ₑ-[NR₇-(A')_{f}-[O-(A")]_{g}-[NR₈-(A‴)]ₕ-Si(R₆')_{d'}(OR₅')_{c'} (II),
- R5, R5', R5" unabhängig voneinander für ein Wasserstoffatom oder für eine C₁-C₆-Alkylgruppe stehen,
- R6, R6' und R6" unabhängig voneinander für eine C₁-C₆-Alkylgruppe stehen,
- A, A', A", A‴ und A′‴ unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen
-
(A′‴)-Si(R₆")_{d}"(OR₅")_{c}" (III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Verfahren nach einem der Ansprüche 1 oder 2, **dadurch gekennzeichnet, dass** das Mittel (a) mindestens eine organische Siliciumverbindung der Formel (I) enthält,
R₁R₂N-L-Si(OR₃)ₐ(R₄)_{b} (I),
wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- R₄ für eine Methylgruppe oder für eine Ethylgruppe steht,
- a für die Zahl 3 steht und
- b für die Zahl 0 steht.

4. Verfahren nach Anspruch 1, **dadurch gekennzeichnet, dass** das Mittel (a) mindestens eine organische Siliciumverbindung der Formel (I) enthält, die ausgewählt ist aus der Gruppe bestehend aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan,
- 1-(2-Dimethylaminoethyl)silantriol und
- Mischungen daraus.

5. Verfahren nach einem der Ansprüche 2 bis 4, **dadurch gekennzeichnet, dass** das Mittel (a) mindestens eine organische Siliciumverbindung der Formel (II) enthält,
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

6. Verfahren nach Anspruch 5, **dadurch gekennzeichnet, dass** es (a) mindestens eine organische Siliciumverbindung der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin,
- N,N-Bis[3-(triethoxysilyl)propyl]-2-Propen-1-amin und
- Mischungen daraus.

7. Verfahren nach einem der Ansprüche 1 bis 6, **dadurch gekennzeichnet, dass** das Mittel (a) mindestens eine organische Siliciumverbindung der Formel (IV) enthält, die ausgewählt ist aus der Gruppe bestehend aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
- Octadecyltrimethoxysilan,
- Octadecyltriethoxysilan und
- Mischungen daraus.

8. Verfahren nach einem der Ansprüche 1 bis 7, **dadurch gekennzeichnet, dass** das Substratplättchen Aluminium umfasst.

9. Verfahren nach einem der Ansprüche 1 bis 8, **dadurch gekennzeichnet, dass** die Metalloxid(hydrat)schicht Siliciumdioxid, vorzugsweise amorphes Siliciumdioxid, umfasst.

10. Verfahren nach einem der Ansprüche 1 bis 9, **dadurch gekennzeichnet, dass** die Metallpartikelträgerschicht wenigstens ein Element ausgewählt aus Sn, Pd, Pt und Au aufweist und/oder die Metallpartikel wenigstens ein Element ausgewählt aus Cu, Ni und Ag aufweisen.

11. Verfahren nach einem der Ansprüche 1 bis 10, **dadurch gekennzeichnet, dass** das Mittel (a) ferner mindestens eine farbgebende Verbindung (a2) enthält, welche vorzugsweise ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

12. Mehrkomponenten-Verpackungseinheit (Kit-of-parts) zum Färben von keratinischem Material, umfassend getrennt voneinander konfektioniert
- einen ersten Container mit einem Mittel (a), wobei das Mittel (a) enthält mindestens eine organische Siliciumverbindungen der Formel (I) und mindestens eine organische Siliciumverbindungen der Formel (IV), wobei in der organischen Siliciumverbindung der Formel (I)
R₁R₂N-L-Si(OR₃)ₐ(R₄)_{b} (I),
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder für eine C₁-C₆-Alkylgruppe steht,
- R₄ für eine C₁-C₆-Alkylgruppe steht,
- a, für eine ganze Zahl von 1 bis 3 steht, und
- b für die ganze Zahl 3 - a steht, und
wobei in der organischen Siliciumverbindung der Formel (IV)
R₉Si(OR₁₀)ₖ(R₁₁)ₘ (IV),
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht, und
- einen zweiten Container mit einem Mittel (b), wobei das Mittel (b) enthält:
(b1) mindestens eine farbgebende Verbindung, umfassend mindestens ein Effektpigment, umfassend i) ein Substratplättchen, ii) eine Metalloxid(hydrat)schicht, welche auf der Oberfläche des Substratplättchens vorgesehen ist, iii) eine Metallpartikelträgerschicht, welche auf der Oberfläche der Metalloxid(hydrat)schicht vorgesehen ist, und iv) Metallpartikel, welche auf der Oberfläche der Metallpartikelträgerschicht vorgesehen sind, und
(b2) mindestens ein filmbildendes Polymer.

## Claims

1. A method for dyeing keratinous material, in particular human hair, comprising the following steps:
- applying an agent (a) to the keratinous material, wherein the agent (a) contains: at least one organosilicon compound of formula (I) and at least one organosilicon compound of formula (IV), where, in the organosilicon compound of formula (I)
R₁R₂N-L-Si(OR₃)ₐ(R₄)_{b} (I),
- R₁ and R₂ represent, independently of one another, a hydrogen atom or a C₁-C₆ alkyl group,
- L represents a linear or branched, divalent C₁-C₂₀ alkylene group,
- R₃ represents a hydrogen atom or a C₁-C₆ alkyl group,
- R₄ represents a C₁-C₆ alkyl group,
- a represents an integer from 1 to 3, and
- b represents the integer 3 - a, and
where, in the organosilicon compound of formula (IV)
R₉Si(OR₁₀)ₖ(R₁₁)ₘ (IV),
- R₉ represents a C₁-C₁₈ alkyl group,
- R₁₀ represents a hydrogen atom or a C₁-C₆ alkyl group,
- R₁₁ represents a C₁-C₆ alkyl group
- k represents an integer from 1 to 3, and
- m represents the integer 3 - k, and
- applying an agent (b) to the keratinous material, wherein the agent (b) contains:
(b1) at least one coloring compound comprising at least one effect pigment, comprising i) a substrate platelet, ii) a metal oxide (hydrate) layer provided on the surface of the substrate platelet, iii) a metal particle carrier layer provided on the surface of the metal oxide (hydrate) layer, and iv) metal particles provided on the surface of the metal particle carrier layer, and
(b2) at least one film-forming polymer.

2. The method according to claim 1, **characterized in that** the agent (a) further contains at least one organosilicon compound of formula (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R5, R5', R5" represent, independently of one another, a hydrogen atom or a C₁-C₆ alkyl group,
- R6, R6' and R6" represent, independently of one another, a C₁-C₆ alkyl group,
- A, A', A", A‴ and A′‴ represent, independently of one another, a linear or branched, divalent C₁-C₂₀ alkylene group,
- R₇ and R₈ represent, independently of one another, a hydrogen atom, a C₁-C₆ alkyl group, a hydroxy C₁-C₆ alkyl group, a C₂-C₆ alkenyl group, an amino C₁-C₆ alkyl group, or a group of formula (III)
-
(A′‴)-Si(R₆ʺ)_{d}ʺ(OR₅ʺ)_{c}'' (III),
- c, represents an integer from 1 to 3,
- d represents the integer 3 - c,
- c' represents an integer from 1 to 3,
- d' represents the integer 3 - c',
- c" represents an integer from 1 to 3,
- d" represents the integer 3 - c",
- e represents 0 or 1,
- f represents 0 or 1,
- g represents 0 or 1,
- h represents 0 or 1,
with the proviso that at least one of the functional groups e, f, g and h is different from 0.

3. The method according to one of claims 1 or 2, **characterized in that** the agent (a)
contains at least one organosilicon compound of formula (I),
R₁R₂N-L-Si(OR₃)ₐ(R₄)_{b} (I),
where
- R₁ and R₂ both represent a hydrogen atom, and
- L represents a linear, divalent C₁-C₆ alkylene group, preferably a propylene group (-CH₂-CH₂-CH₂-) or an ethylene group (-CH₂-CH₂-),
- R₃ represents a hydrogen atom, an ethyl group or a methyl group,
- R₄ represents a methyl group or an ethyl group,
- a represents the number 3, and
- b represents the number 0.

4. The method according to claim 1, **characterized in that** the agent (a)
contains at least one organosilicon compound of formula (I) which is selected from the group consisting of
- (3-aminopropyl)triethoxysilane
- (3-aminopropyl)trimethoxysilane
- 1-(3-aminopropyl)silanetriol
- (2-aminoethyl)triethoxysilane
- (2-aminoethyl)trimethoxysilane
- 1-(2-aminoethyl)silanetriol
- (3-dimethylaminopropyl)triethoxysilane
- (3-dimethylaminopropyl)trimethoxysilane
- 1-(3-dimethylaminopropyl)silanetriol
- (2-dimethylaminoethyl)triethoxysilane
- (2-dimethylaminoethyl)trimethoxysilane,
- 1-(2-dimethylaminoethyl)silanetriol, and
- mixtures thereof.

5. The method according to one of claims 2 to 4, **characterized in that** the agent (a) contains at least one organosilicon compound of formula (II),
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
where
- e and f both represent the number 1,
- g and h both represent the number 0,
- A and A' represent, independently of one another, a linear, divalent C₁-C₆ alkylene group, and
- R7 represents a hydrogen atom, a methyl group, a 2-hydroxyethyl group, a 2-alkenyl group, a 2-aminoethyl group or a group of formula (III).

6. The method according to claim 5, **characterized in that** (a) contains at least one organosilicon compound of formula (II) which is selected from the group consisting of
- 3-(trimethoxysilyl)-N-[3-(trimethoxysilyl)propyl]-1-propanamine
- 3-(triethoxysilyl)-N-[3-(triethoxysilyl)propyl]-1-propanamine
- N-methyl-3-(trimethoxysilyl)-N-[3-(trimethoxysilyl)propyl]-1-propanamine
- N-methyl-3-(triethoxysilyl)-N-[3-(triethoxysilyl)propyl]-1-propanamine
- 2-[bis[3-(trimethoxysilyl)propyl]amino]ethanol
- 2-[bis[3-(triethoxysilyl)propyl]amino]ethanol
- 3-(trimethoxysilyl)-N,N-bis[3-(trimethoxysilyl)propyl]-1-propanamine
- 3-(triethoxysilyl)-N,N-bis[3-(triethoxysilyl)propyl]-1-propanamine
- N1,N1-bis[3-(trimethoxysilyl)propyl]-1,2-ethanediamine,
- N1,N1-bis[3-(triethoxysilyl)propyl]-1,2-ethanediamine,
- N,N-bis[3-(triethoxysilyl)propyl]-2-propen-1-amine,
- N,N-bis[3-(trimethoxysilyl)propyl]-2-propen-1-amine, and
- mixtures thereof.

7. The method according to one of claims 1 to 6, **characterized in that** the agent (a) contains at least one organosilicon compound of formula (IV) which is selected from the group consisting of
- methyltrimethoxysilane
- methyltriethoxysilane
- ethyltrimethoxysilane
- ethyltriethoxysilane
- propyltrimethoxysilane
- propyltriethoxysilane
- hexyltrimethoxysilane
- hexyltriethoxysilane
- octyltrimethoxysilane
- octyltriethoxysilane
- dodecyltrimethoxysilane,
- dodecyltriethoxysilane,
- octadecyltrimethoxysilane,
- octadecyltriethoxysilane, and
- mixtures thereof.

8. The method according to one of claims 1 to 7, **characterized in that** the substrate platelet comprises aluminum.

9. Method according to one of claims 1 to 8, **characterized in that** the metal oxide (hydrate) layer comprises silicon dioxide, preferably amorphous silicon dioxide.

10. Method according to one of claims 1 to 9, **characterized in that** the metal particle carrier layer contains at least one element selected from Sn, Pd, Pt and Au and/or the metal particles contain at least one element selected from Cu, Ni and Ag.

11. The method according to one of claims 1 to 10, **characterized in that** the agent (a) further contains at least one coloring compound (a2), which is preferably selected from the group of colored metal oxides, metal hydroxides, metal oxide hydrates, silicates, metal sulfides, complex metal cyanides, metal sulfates, bronze pigments and/or from mica-based colored pigments which are coated with at least one metal oxide and/or metal oxychloride.

12. A multi-component packaging unit (kit-of-parts) for dyeing keratinous material, comprising, packaged separately from one another,
- a first container having an agent (a), wherein the agent (a) contains at least one organosilicon compound of formula (I) and at least one organosilicon compound of formula (IV), where, in the organosilicon compound of formula (I)
R₁R₂N-L-Si(OR₃)ₐ(R₄)_{b} (I),
- R₁ and R₂ represent, independently of one another, a hydrogen atom or a C₁-C₆ alkyl group,
- L represents a linear or branched, divalent C₁-C₂₀ alkylene group,
- R₃ represents a hydrogen atom or a C₁-C₆ alkyl group,
- R₄ represents a C₁-C₆ alkyl group,
- a represents an integer from 1 to 3, and
- b represents the integer 3 - a, and
where, in the organosilicon compound of formula (IV)
R₉Si(OR₁₀)ₖ(R₁₁)ₘ (IV),
- R₉ represents a C₁-C₁₈ alkyl group,
- R₁₀ represents a hydrogen atom or a C₁-C₆ alkyl group,
- R₁₁ represents a C₁-C₆ alkyl group
- k represents an integer from 1 to 3, and
- m represents the integer 3 - k, and
- a second container having an agent (b), wherein the agent (b) contains:
(b1) at least one coloring compound comprising at least one effect pigment, comprising i) a substrate platelet, ii) a metal oxide (hydrate) layer provided on the surface of the substrate platelet, iii) a metal particle carrier layer provided on the surface of the metal oxide (hydrate) layer, and iv) metal particles provided on the surface of the metal particle carrier layer, and
(b2) at least one film-forming polymer.

## Revendications

1. Procédé permettant la coloration de matière kératinique, en particulier de cheveux humains, comprenant les étapes suivantes :
- application d'un agent (a) sur la matière kératinique, dans lequel l'agent (a) contient : au moins un composé organique de silicium de formule (I) et au moins un composé organique de silicium de formule (IV), où, dans le composé organique de silicium de formule (I)
R₁R₂N-L-Si(OR₃)ₐ(R₄)_{b} (1),
- R₁, R₂ représentent, indépendamment l'un de l'autre, un atome d'hydrogène ou un groupe alkyle en C₁-C₆,
- L représente un groupe alkylène en C₁-C₂₀ divalent, linéaire ou ramifié,
- R₃ représente un atome d'hydrogène ou un groupe alkyle en C₁-C₆,
- R₄ représente un groupe alkyle en C₁-C₆,
- a, représente un nombre entier allant de 1 à 3, et
- b représente le nombre entier 3 - a, et
où, dans le composé organique de silicium de formule (IV)
R₉Si(OR₁₀)ₖ(R₁₁)ₘ (IV),
- R₉ représente un groupe alkyle en C₁-C₁₈,
- R₁₀ représente un atome d'hydrogène ou un groupe alkyle en C₁-C₆,
- R₁₁ représente un groupe alkyle en C₁-C₆
- k représente un nombre entier allant de 1 à 3, et
- m représente le nombre entier 3 - k, et
- application d'un agent (b) sur la matière kératinique, dans lequel l'agent (b) contient :
(b1) au moins un composé colorant comprenant au moins un pigment à effet, comprenant i) une plaquette substrat, ii) une couche d'oxyde métallique (hydraté) prévue sur la surface de la plaquette substrat, iii) une couche de support de particules métalliques prévue sur la surface de la couche d'oxyde métallique (hydraté), et iv) des particules métalliques prévues sur la surface de la couche de support de particules métalliques, et
(b2) au moins un polymère filmogène.

2. Procédé selon la revendication 1, **caractérisé en ce que** l'agent (a) contient en outre au moins un composé organique de silicium de formule (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R5, R5', R5" représentent, indépendamment les uns des autres, un atome d'hydrogène ou un groupe alkyle en C₁-C₆,
- R6, R6' et R6" représentent, indépendamment les uns des autres, un groupe alkyle en C₁-C₆,
- A, A', A", A‴ et A′‴ représentent, indépendamment les uns des autres, un groupe alkylène en C₁-C₂₀ divalent, linéaire ou ramifié,
- R₇ et R₈ représentent, indépendamment l'un de l'autre, un atome d'hydrogène, un groupe alkyle en C₁-C₆, un groupe hydroxy alkyle en C₁-C₆, un groupe alcényle en C₂-C₆, un groupe amino alkyle en C₁-C₆ ou un groupement de formule (III)
-(A′‴)-Si(R₆")_{d}"(OR₅")_{c}" (III),
- c, représente un nombre entier allant de 1 à 3,
- d représente le nombre entier 3 - c,
- c' représente un nombre entier allant de 1 à 3,
- d' représente le nombre entier 3 - c',
- c" représente un nombre entier allant de 1 à 3,
- d" représente le nombre entier 3 - c",
- e représente 0 ou 1,
- f représente 0 ou 1,
- g représente 0 ou 1,
- h représente 0 ou 1,
à condition qu'au moins l'un des radicaux parmi e, f, g et h soit différent de 0.

3. Procédé selon l'une des revendications 1 ou 2, **caractérisé en ce que** l'agent (a) contient au moins un composé organique de silicium de formule (I)
R₁R₂N-L-Si(OR₃)ₐ(R₄)_{b} (I),
où
- R₁, R₂ représentent tous deux un atome d'hydrogène, et
- L représente un groupe alkylène en C₁-C₆ divalent linéaire, de préférence un groupe propylène (-CH₂-CH₂-CH₂-) ou un groupe éthylène (-CH₂-CH₂-),
- R₃ représente un atome d'hydrogène, un groupe éthyle ou un groupe méthyle,
- R₄ représente un groupe méthyle ou un groupe éthyle,
- a représente le nombre 3, et
- b représente le nombre 0.

4. Procédé selon la revendication 1, **caractérisé en ce que** l'agent (a) contient au moins un composé organique de silicium de formule (I) qui est choisi dans le groupe constitué de
- (3-aminopropyl)triéthoxysilane
- (3-aminopropyl)triméthoxysilane
- 1-(3-aminopropyl)silanetriol
- (2-aminoéthyl)triéthoxysilane
- (2-aminoéthyl)triméthoxysilane
- 1-(2-aminoéthyl)silanetriol
- (3-diméthylaminopropyl)triéthoxysilane
- (3-diméthylaminopropyl)triméthoxysilane
- 1-(3-diméthylaminopropyl)silanetriol
- (2-diméthylaminoéthyl)triéthoxysilane
- (2-diméthylaminoéthyl)triméthoxysilane,
- 1-(2-diméthylaminoéthyl)silanetriol, et
- mélanges de ceux-ci.

5. Procédé selon l'une des revendications 2 à 4, **caractérisé en ce que** l'agent (a) contient au moins un composé organique de silicium de formule (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
où
- e et f représentent tous deux le nombre 1,
- g et h représentent tous deux le nombre 0,
- A et A' représentent, indépendamment l'un de l'autre, un groupe alkylène en C₁-C₆ divalent linéaire, et
- R7 représente un atome d'hydrogène, un groupe méthyle, un groupe 2-hydroxyéthyle, un groupe 2-alcényle, un groupe 2-aminoéthyle ou un groupement de formule (III).

6. Procédé selon la revendication 5, **caractérisé en ce qu'**il (a) contient au moins un composé organique de silicium de formule (II) qui est choisi dans le groupe constitué de
- 3-(triméthoxysilyl)-N-[3-(triméthoxysilyl)propyl]-1-propanamine
- 3-(triéthoxysilyl)-N-[3-(triéthoxysilyl)propyl]-1-propanamine
- N-méthyl-3-(triméthoxysilyl)-N-[3-(triméthoxysilyl)propyl]-1-propanamine
- N-méthyl-3-(triéthoxysilyl)-N-[3-(triéthoxysilyl)propyl]-1-propanamine
- 2-[bis[3-(triméthoxysilyl)propyl]amino]-éthanol
- 2-[bis[3-(triéthoxysilyl)propyl]amino]-éthanol
- 3-(triméthoxysilyl)-N,N-bis[3-(triméthoxysilyl)propyl]-1-propanamine
- 3-(triéthoxysilyl)-N,N-bis[3-(triéthoxysilyl)propyl]-1-propanamine
- N1,N1-bis[3-(triméthoxysilyl)propyl]-1,2-éthanediamine,
- N1,N1-bis[3-(triéthoxysilyl)propyl]-1,2-éthanediamine,
- N,N-bis[3-(triméthoxysilyl)propyl]-2-propén-1-amine,
- N,N-bis[3-(triéthoxysilyl)propyl]-2-propén-1-amine, et
- mélanges de ceux-ci.

7. Procédé selon l'une des revendications 1 à 6, **caractérisé en ce que** l'agent (a) contient au moins un composé organique de silicium de formule (IV) qui est choisi dans le groupe constitué de
- méthyltriméthoxysilane
- méthyltriéthoxysilane
- éthyltriméthoxysilane
- éthyltriéthoxysilane
- propyltriméthoxysilane
- propyltriéthoxysilane
- hexyltriméthoxysilane
- hexyltriéthoxysilane
- octyltriméthoxysilane
- octyltriéthoxysilane
- dodécyltriméthoxysilane,
- dodécyltriéthoxysilane,
- octadécyltriméthoxysilane,
- octadécyltriéthoxysilane, et
- mélanges de ceux-ci.

8. Procédé selon l'une des revendications 1 à 7, **caractérisé en ce que** la plaquette substrat comprend de l'aluminium.

9. Procédé selon l'une des revendications 1 à 8, **caractérisé en ce que** la couche d'oxyde métallique (hydraté) comprend de la silice, de préférence de la silice amorphe.

10. Procédé selon l'une des revendications 1 à 9, **caractérisé en ce que** la couche de support de particules métalliques présente au moins un élément choisi parmi Sn, Pd, Pt et Au et/ou les particules métalliques présentent au moins un élément choisi parmi Cu, Ni et Ag.

11. Procédé selon l'une des revendications 1 à 10, **caractérisé en ce que** l'agent (a) contient en outre au moins un composé colorant (a2) qui est de préférence choisi dans le groupe des oxydes métalliques colorés, hydroxydes métalliques colorés, oxydes métalliques hydratés colorés, silicates colorés, sulfures métalliques colorés, cyanures métalliques complexes colorés, sulfates métalliques colorés, pigments de bronze colorés, et/ou des pigments colorés à base de mica revêtus d'au moins un oxyde métallique et/ou un oxychlorure métallique.

12. Unité d'emballage à plusieurs composants (kit de pièces) permettant la coloration d'une matière kératinique, comprenant, conditionnés de manière à être séparés les uns des autres,
- un premier récipient comportant un agent (a), dans laquelle l'agent (a) contient au moins un composé organique de silicium de formule (I) et au moins un composé organique de silicium de formule (IV), où, dans le composé organique de silicium de formule (I)
R₁R₂N-L-Si(OR₃)ₐ(R₄)_{b} (1),
- R₁, R₂ représentent, indépendamment l'un de l'autre, un atome d'hydrogène ou un groupe alkyle en C₁-C₆,
- L représente un groupe alkylène en C₁-C₂₀ divalent, linéaire ou ramifié,
- R₃ représente un atome d'hydrogène ou un groupe alkyle en C₁-C₆,
- R₄ représente un groupe alkyle en C₁-C₆,
- a, représente un nombre entier allant de 1 à 3, et
- b représente le nombre entier 3 - a, et
où, dans le composé organique de silicium de formule (IV)
R₉Si(OR₁₀)ₖ(R₁₁)ₘ (IV),
- R₉ représente un groupe alkyle en C₁-C₁₈,
- R₁₀ représente un atome d'hydrogène ou un groupe alkyle en C₁-C₆,
- R₁₁ représente un groupe alkyle en C₁-C₆
- k représente un nombre entier allant de 1 à 3, et
- m représente le nombre entier 3 - k, et
- un second récipient comportant un agent (b), dans laquelle l'agent (b) contient :
(b1) au moins un composé colorant comprenant au moins un pigment à effet, comprenant i) une plaquette substrat, ii) une couche d'oxyde métallique (hydraté) prévue sur la surface de la plaquette substrat, iii) une couche de support de particules métalliques prévue sur la surface de la couche d'oxyde métallique (hydraté), et iv) des particules métalliques prévues sur la surface de la couche de support de particules métalliques, et
(b2) au moins un polymère filmogène.
